# EUROPÄISCHE PATENTSCHRIFT

(11) **EP 1 751 155 B1**
(45) Veröffentlichungstag und Bekanntmachung des Hinweises auf die Patenterteilung: **07.01.2009**
(21) Anmeldenummer: 05747551.9
(22) Anmeldetag: 23.04.2005
(51) Int. Cl.: C07D 471/04, A61K 31/519, A61P 31/22

(54) **SUBSTITUIERTE AZACHINAZOLINE MIT ANTIVIRALER WIRKUNG**
SUBSTITUTED AZACHINAZOLINES HAVING AN ANTIVIRAL ACTION
AZACHINAZOLINES SUBSTITUEES A ACTION ANTIVIRALE

(30) Priorität: 07.05.2004 DE 102004022672
(43) Veröffentlichungstag der Anmeldung: 14.02.2007
(73) Patentinhaber: Bayer HealthCare AG, 51368 Leverkusen (DE)
(72) Erfinder: WUNBERG, Tobias, 2371 Hinterbrühl (AT); BAUMEISTER, Judith, 2800 Melchelen (BE); JESKE, Mario, 42699 Solingen (DE); SÜSSMEIER, Frank, 42277 Wuppertal (DE); ZIMMERMANN, Holger, 42113 Wuppertal (DE); HENNINGER, Kerstin, 42115 Wuppertal (DE); LANG, Dieter, 42553 Velbert (DE)
(86) Internationale Anmeldenummer: PCT/EP2005/004384
(87) Internationale Veröffentlichungsnummer: WO 2005/113552

(56) Entgegenhaltungen:
- EP-A- 1 201 765
- WO-A-20/04072048
- WO-A-20/04096778
- WO-A-20/04099212
- US-A1- 2002 019 397
- T. SAITO ET. AL.: "A Facile and Efficient Carbodiimide Mediated Synthesis of Dihydroquinazolines via a Tandem Nucleophilic Addition-Intramolecular Hetero conjugate Addition Annulation Strategy." TETRAHEDRON LETTERS, Bd. 37, Nr. 2, 1996, Seiten 209-212, XP004030439 in der Anmeldung erwähnt
- F. WANG ET. AL.: "Solid Phase Synthesis of 3,4-Dihydroquinazoline" TETRAHEDRON LETTERS, Bd. 38, Nr. 50, 1997, Seiten 8651-4, XP004097142 in der Anmeldung erwähnt

## Beschreibung

Die Erfindung betrifft substituierte Azachinazoline und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

Die Synthese von Dihydrochinazolinen ist beschrieben in Saito T., et al. Tetrahedron Lett., 1996, 37, 209-212 und in Wang F., et al. Tetrahedron Lett., 1997, 38, 8651-8654.

EP-A-1,201,765 offenbart unter anderem substituierte Phenyl-(chinazolin-4-yl)-amine, substituierte (3-Morpholin-4-yl-propylamino)-8*H*-pyrido[2,3-*d*]pyrimidin-7-one und substituierte (Pyridin-4-ylamino)-8*H*-pyrido[2,3-*d*]pyrimidin-7-one und ihre Verwendung zur Bekämpfung von Cytomegaloviren. US-A-2002019397 offenbart unter anderem substituierte N-Benzyl-naphthyridincarboxamide und ihre Verwendung gegen Cytomegaloviren.

Auf dem Markt sind zwar strukturell andersartige antiviral wirkende Mittel vorhanden, aber die gegenwärtig verfügbaren Therapien mit Ganciclovir, Valganciclovir, Foscarnet und Cidofovir sind mit schweren Nebenwirkungen verbunden, z.B. Nephrotoxizität, Neutropenie oder Thrombozytopenie. Zudem kann es regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Azachinazoline antiviral wirksam sind.

Gegenstand der Erfindung sind Verbindungen der Formel 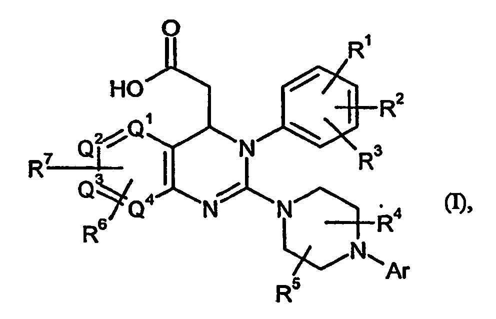 in welcher
- Ar: für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Formyl, Hydroxycarbonyl, Alkylcarbonyl, Alkoxycarbonyl, Trifluormethyl, Halogen, Cyano, Hydroxy, Amino, Alkylamino, Aminocarbonyl und Nitro, worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,
oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls vorhandener dritter Substituent unabhängig davon aus der genannten Gruppe ausgewählt wird,
- Q¹, Q², Q³ und Q⁴: für CH oder N stehen,
wobei ein oder zwei von Q¹, Q², Q³ und Q⁴ für N stehen und die anderen gleichzeitig für CH stehen,
- R¹: für Wasserstoff, Amino, Alkyl, Alkoxy, Alkylamino, Alkylthio, Cyano, Halogen, Nitro oder Trifluormethyl steht,
- R²: für Wasserstoff, Alkyl, Alkoxy, Alkylthio, Cyano, Halogen, Nitro oder Trifluormethyl steht,
- R³: für Amino, Alkyl, Alkoxy, Alkylamino, Alkylthio, Cyano, Halogen, Nitro, Trifluormethyl, Alkylsulfonyl oder Alkylaminosulfonyl steht
oder
einer der Reste R¹, R² und R³ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,
- R⁴: für Wasserstoff oder Alkyl steht,
- R⁵: für Wasserstoff oder Alkyl steht
oder
die Reste R⁴ und R⁵ im Piperazin-Ring an genau gegenüberliegenden Kohlenstoffatomen gebunden sind und eine gegebenenfalls mit 1 bis 2 Methylgruppen substituierte Methylen-Brücke bilden,
- R⁶: für Wasserstoff, Alkyl, Alkoxy, Alkylthio, Formyl, Hydroxycarbonyl, Aminocarbonyl, Alkylcarbonyl, Alkoxycarbonyl, Trifluormethyl, Halogen, Cyano, Hydroxy oder Nitro steht
und
- R⁷: für Wasserstoff, Alkyl, Alkoxy, Alkylthio, Formyl, Hydroxycarbonyl, Alkylcarbonyl, Alkoxycarbonyl, Trifluormethyl, Halogen, Cyano, Hydroxy oder Nitro steht,
und ihre Salze, ihre Solvate und die Solvate ihrer Salze.

Erfindungsgemäße Verbindungen sind die Verbindungen der Formel (I) und deren Salze, Solvate und Solvate der Salze, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

### Alkyl per se und "Alk" und "Alkyl" in Alkoxy, Alkylthio, Alkylamino, Alkylcarbonyl, Alkylsulfonyl,

Alkylaminosulfonyl und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, n-Pentyl und n-Hexyl.

Alkoxy steht beispielhaft und vorzugsweise für Methoxy, Ethoxy, n-Propoxy, Isopropoxy, *tert-*Butoxy, n-Pentoxy und n-Hexoxy.

Alkylthio steht beispielhaft und vorzugsweise für Methylthio, Ethylthio, n-Propylthio, Isopropylthio, *tert*-Butylthio, n-Pentylthio und n-Hexylthio.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Butylamino, n-Pentylamino, n-Hexylamino, *N*,*N*-Dimethylamino, *N*,*N*-Diethylamino, *N*-Ethyl-*N*-methylamino, *N*-Methyl-*N*-n-propylamino, *N*-Isopropyl-*N*-n-propylamino, *N*-*tert*-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylsulfonyl steht beispielhaft und vorzugsweise für Methylsulfonyl, Ethylsulfonyl, n-Propylsulfonyl, Isopropylsulfonyl, *tert*-Butylsulfonyl, n-Pentylsulfonyl und n-Hexylsulfonyl.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, Isopropylaminosulfonyl, *tert*-Butylaminosulfonyl, n-Pentylaminosulfonyl, n-Hexyl-aminosulfonyl, *N*,*N*-Dimethylaminosulfonyl, *N*,*N*-Diethylaminosulfonyl, *N*-Ethyl-*N*-methylaminosulfonyl, *N*-Methyl-*N*-n-propylaminosulfonyl, *N*-Isopropyl-*N-*n-propyl-aminosulfonyl, *N*-*tert*-Butyl-*N*-methylaminosulfonyl, *N*-Ethyl-*N*-n-pentylamino-sulfonyl und *N*-n-Hexyl-*N*-methylaminosulfonyl. C₁-C₃-Alkylaminosulfonyl steht beispielsweise für einen Monoalkylaminosulfonylrest mit 1 bis 3 Kohlenstoffatomen oder für einen Dialkylaminosulfonylrest mit jeweils 1 bis 3 Kohlenstoffatomen pro Alkylsubstituent.

Alkylcarbonyl steht beispielhaft und vorzugsweise für Acetyl und Propanoyl.

Alkoxycarbonyl steht beispielhaft und vorzugsweise für Methoxycarbonyl, Ethoxycarbonyl, n-Propoxycarbonyl, Isopropoaycarbonyl, *tert*-Butoxycarbonyl, n-Pentoxycarbonyl und n-Hexoxycarbonyl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Halogen steht für Fluor, Chlor, Brom und Jod, bevorzugt Fluor und Chlor.

Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90% verstanden wird (> 90% ee).

Bevorzugt sind solche Verbindungen der Formel (I), in welchen
- Ar: für Phenyl steht, worin Phenyl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Hydroxycarbonyl, C₁-C₆-Alkylcarbonyl, C₁-C₆-Alkoxycarbonyl, Trifluormethyl, Fluor, Chlor, Brom, Cyano, Hydroxy, Amino, C₁-C₆-Alkylamino und Nitro,
oder zwei der Substituenten am Phenyl zusammen mit den Kohlenstoffatomen, an die sie gebunden sind, ein 1,3-Dioxolan bilden und ein gegebenenfalls vorhandener dritter Substituent unabhängig davon aus der genannten Gruppe ausgewählt wird,
- Q¹, Q² und Q³: für CH oder N stehen,
wobei immer genau einer von Q¹, Q² und Q³ für N steht und die anderen gleichzeitig für CH stehen,
- Q⁴: für CH steht,
- R¹: für Wasserstoff, C₁-C₃-Alkyl, C₁-C₃-Alkoxy, C₁-C₃-Alkylthio, Fluor oder Chlor steht,
- R²: für Wasserstoff, C₁-C₃-Alkyl, C₁-C₃-Alkoxy, C₁-C₃-Alkylthio, Fluor oder Chlor steht,
- R³: für C₁-C₄-Alkyl, Cyano, Fluor, Chlor, Nitro, Trifluormethyl oder C₁-C₃-Alkylsulfonyl steht,
oder
einer der Reste R¹, R² und R³ für Wasserstoff, C₁-C₃-Alkyl, C₁-C₃-Alkoxy, Cyano, Halogen, Nitro oder Trifluormethyl steht und die anderen beiden zusammen mit den Kohlenstoffatomen, an die sie gebunden sind, einen Cyclopentan-Ring oder einen Cyclohexan-Ring bilden,
- R⁴: für Wasserstoff oder Methyl steht,
- R⁵: für Wasserstoff steht,
- R⁶: für Wasserstoff, C₁-C₃-Alkyl, C₁-C₃-Alkoxy, Hydroxycarbonyl, Aminocarbonyl, Trifluormethyl, Fluor, Chlor, Cyano, Hydroxy oder Nitro steht
und
- R⁷: für Wasserstoff, C₁-C₃-Alkyl, C₁-C₃-Alkoxy, Fluor, Chlor, Cyano oder Hydroxy steht.

Bevorzugt sind darunter besonders solche Verbindungen der Formel (I), in welchen
- Ar: für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
- Q¹, Q² und Q³: für CH oder N stehen,
wobei immer genau einer von Q¹, Q² und Q³ für N steht und die anderen gleichzeitig für CH stehen,
- Q⁴: für CH steht,
- R¹: für Wasserstoff, Methyl, Methoxy, Methylthio, Fluor oder Chlor steht,
- R²: für Wasserstoff steht,
- R³: für Methyl, iso-Propyl, *tert*-Butyl, Cyano, Fluor, Chlor, Nitro oder Trifluormethyl steht,
- R⁴: für Wasserstoff steht,
- R⁵: für Wasserstoff steht,
- R⁶: für Wasserstoff, Aminocarbonyl, Fluor, Chlor, Cyano oder Hydroxy steht
und
- R⁷: für Wasserstoff steht.

Bevorzugt sind darunter ganz besonders solche Verbindungen der Formel (I), in welchen
- Ar: für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
- Q¹, Q² und Q³: für CH oder N stehen,
wobei immer genau einer von Q¹, Q² und Q³ für N steht und die anderen gleichzeitig für CH stehen,
- Q⁴: für CH steht,
- R¹: für Wasserstoff, Methyl oder Methoxy steht,
- R²: für Wasserstoff steht,
- R³: für Methyl, *tert*-Butyl, Chlor oder Trifluormethyl steht,
- R⁴: für Wasserstoff steht,
- R⁵: für Wasserstoff steht,
- R⁶: für Wasserstoff steht
und
- R⁷: für Wasserstoff steht.

Bevorzugt sind auch solche Verbindungen der Formel (I), in welchen R¹ für Wasserstoff, Methyl, Methoxy oder Fluor steht.

Bevorzugt sind darunter besonders solche Verbindungen der Formel (I), in welchen R¹ für Methoxy steht.

Bevorzugt sind auch solche Verbindungen der Formel (I), in welchen R¹ über die ortho-Position zur Verknüpfungsstelle des Phenylrings an den Phenylring gebunden ist. Unter der Verknüpfungsstelle des mit den Resten R¹, R² und R³ substituierten Phenylrings wird im Rahmen der vorliegenden Erfindung das gemäß Formel (I) mit einem der beiden Dihydrochinazolin-Stickstoffatome verknüpfte Kohlenstoffatom des Phenylrings verstanden.

Besonders bevorzugt sind solche Verbindungen der Formel (I), in welchen R¹ für Methoxy steht und R¹ über die ortho-Position zur Verknüpfungsstelle des Phenylrings an den Phenylring gebunden ist.

Bevorzugt sind auch solche Verbindungen der Formel (I), in welchen R² für Wasserstoff steht.

Bevorzugt sind auch solche Verbindungen der Formel (I), in welchen R³ für Trifluormethyl, Chlor, Methyl, iso-Propyl oder *tert*-Butyl steht.

Bevorzugt sind darunter besonders solche Verbindungen der Formel (I), in welchen R³ für Trifluormethyl, Chlor oder Methyl steht.

Bevorzugt sind darunter ganz besonders solche Verbindungen der Formel (I), in welchen R³ für Trifluormethyl steht.

Bevorzugt sind auch solche Verbindungen der Formel (I), in welchen R¹ über die ortho-Position zur Verknüpfungsstelle des Phenylrings an den Phenylring gebunden ist und R³ über die R¹ gegenüberliegende meta-Position zur Verknüpfungsstelle des Phenylrings an den Phenylring gebunden ist.

Besonders bevorzugt sind solche Verbindungen der Formel (I), in welchen R¹ über die ortho-Position zur Verknüpfungsstelle des Phenylrings an den Phenylring gebunden ist, R³ für Trifluormethyl, Chlor oder Methyl steht und R³ über die R¹ gegenüberliegende meta-Position zur Verknüpfungsstelle des Phenylrings an den Phenylring gebunden ist.

Besonders bevorzugt sind darunter solche Verbindungen der Formel (I), in welchen R¹ über die ortho-Position zur Verknüpfungsstelle des Phenylrings an den Phenylring gebunden ist, R³ für Trifluormethyl steht und R³ über die R¹ gegenüberliegende meta-Position zur Verknüpfungsstelle des Phenylrings an den Phenylring gebunden ist.

Bevorzugt sind auch solche Verbindungen der Formel (I), in welchen R⁴ und R⁵ für Wasserstoff stehen.

Bevorzugt sind auch solche Verbindungen der Formel (I), in welchen R⁶ für Wasserstoff steht.

Bevorzugt sind auch solche Verbindungen der Formel (I), in welchen R⁷ für Wasserstoff steht.

Bevorzugt sind auch solche Verbindungen der Formel (I), in welchen Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei Verbindungen der Formel 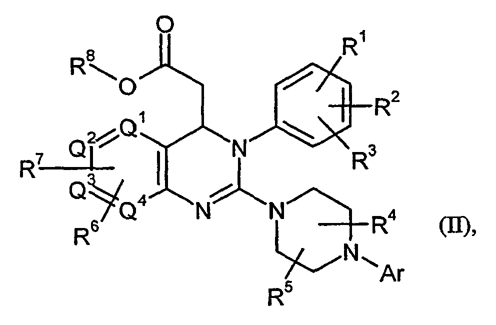 in welcher
Ar, Q¹, Q², Q³, Q⁴, R¹, R², R³, R⁴, R⁵, R⁶ und R⁷ die oben angegebene Bedeutung haben und
R⁸ für Alkyl, bevorzugt für Methyl oder Ethyl oder für *tert*-Butyl, steht,
mit Basen oder Säuren umgesetzt werden.

Die Umsetzung erfolgt im Falle von Methyl und Ethyl im allgemeinen mit Basen in inerten Lösungsmitteln, bevorzugt in einem Temperaturbereich von Raumtemperatur bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, gegebenenfalls in wässriger Lösung, bevorzugt ist Natriumhydroxid in Wasser.

Inerte Lösungsmittel sind beispielsweise Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder *tert*-Butanol, oder Gemischen von Lösungsmitteln, bevorzugt ist Dioxan oder Tetrahydrofuran.

Die Umsetzung erfolgt im Falle von *tert*-Butyl im allgemeinen mit Säuren in inerten Lösungsmitteln, bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Verbindungen der Formel (II) sind bekannt oder können hergestellt werden, indem Verbindungen der Formel 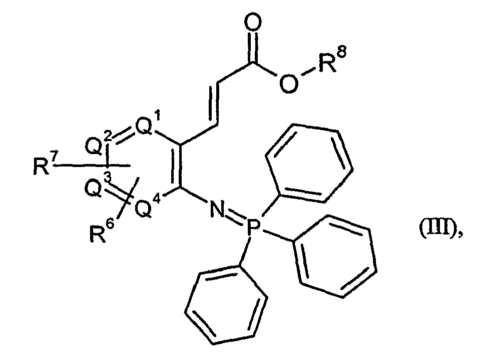 in welcher
Q¹, Q², Q³, Q⁴, R⁶, R⁷ und R⁸ die oben angegebene Bedeutung haben,
in einer zweistufigen Reaktion zunächst mit Verbindungen der Formel 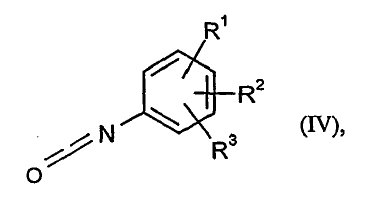 in welcher
R¹, R² und R³ die oben angegebene Bedeutung haben,
und anschließend mit Verbindungen der Formel 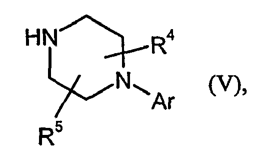 in welcher
Ar, R⁴ und R⁵ die oben angegebene Bedeutung haben,
umgesetzt werden.

Die Umsetzung erfolgt in beiden Stufen im allgemeinen in inerten Lösungsmitteln, bevorzugt in einem Temperaturbereich von Raumtemperatur bis 100°C bei Normaldruck. In der zweiten Stufe wird gegebenenfalls Kieselgel zu der Reaktionsmischung dazugegeben. Die Umsetzung erfolgt bevorzugt mit einer Aufarbeitung zwischen der ersten und der zweiten Stufe.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-*tert*-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Essigsäureethylester, oder Gemischen von Lösungsmitteln, bevorzugt ist Methylenchlorid.

Die Verbindungen der Formel (IV) sind bekannt oder lassen sich nach bekannten Verfahren aus den entsprechenden Edukten synthetisieren.

Die Verbindungen der Formel (V) sind bekannt oder lassen sich nach bekannten Verfahren aus den entsprechenden Edukten synthetisieren, beispielsweise durch eine Buchwald-Hartwig-Reaktion nach folgendem Syntheseschema (Übersicht in: C.G. Frost, P. Mendonca, J. Chem. Soc., Perkin Trans I, 1998, 2615-2623):

### Buchwald-Hartwig-Reaktion:

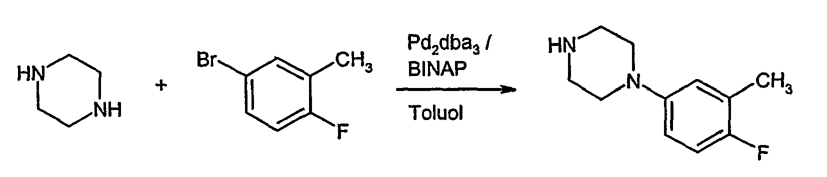

Die dafür benötigten Edukte sind bekannt oder lassen sich nach bekannten Verfahren aus den entsprechenden Edukten synthetisieren.

Die Verbindungen der Formel (III) sind bekannt oder können hergestellt werden, indem Verbindungen der Formel 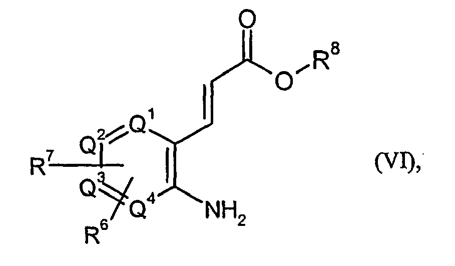 in welcher
Q¹, Q², Q³, Q⁴, R⁶, R⁷ und R⁸ die oben angegebene Bedeutung haben,
mit Triphenylphosphin und Tetrachlorkohlenstoff umgesetzt werden.

Die Umsetzung erfolgt im allgemeinen in inerten Lösungsmitteln, in Gegenwart einer Base, bevorzugt in einem Temperaturbereich von Raumtemperatur bis 50°C bei Normaldruck.

Inerte Lösungsmittel sind beispielsweise Ether wie Diethylether, Methyl-*tert*-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, bevorzugt ist Acetonitril.

Basen sind beispielsweise Alkali- und Erdalkalicarbonate wie Cäsiumcarbonat, Natrium- oder Kaliumcarbonat oder Amine wie Triethylamin, Diisopropylethylamin, *N*-Methylmorpholin oder Pyridin, bevorzugt ist Triethylamin.

Die Verbindungen der Formel (VI) sind bekannt oder lassen sich nach bekannten Verfahren aus den entsprechenden Edukten synthetisieren, beispielsweise durch eine Heck-Reaktion oder eine Wittig-Horner-Reaktion nach folgenden Syntheseschemata:

### Heck-Reaktion:

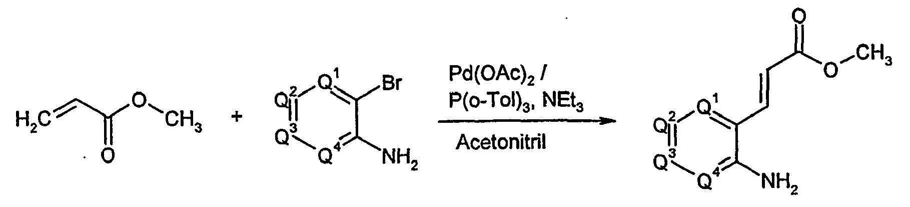

### Wittig-Horner-Reaktion:

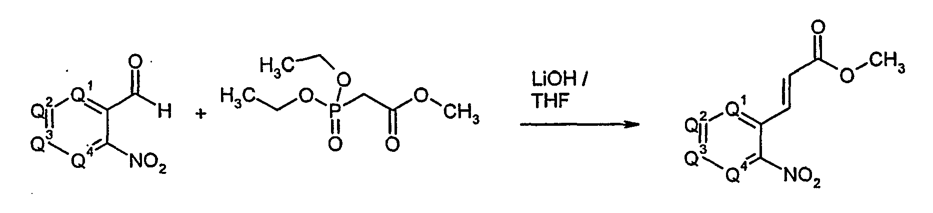

Die dafür benötigten Edukte sind bekannt oder lassen sich nach bekannten Verfahren aus den entsprechenden Edukten synthetisieren.

Die Herstellung der erfindungsgemäßen Verbindungen kann durch das folgende Syntheseschema verdeutlicht werden.

### Syntheseschema:

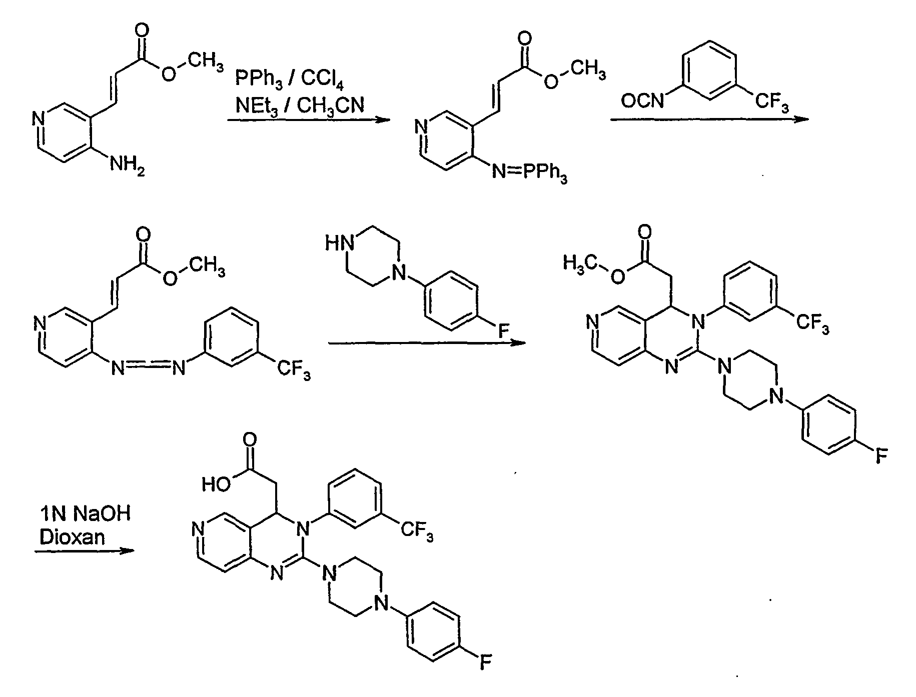

Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und KrebsTherapie.
6) Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antiviral wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch, topisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

| | |
|---|---|
| ca. | circa |
| BINAP | 2,2'-Bis(diphenylphosphino)-1,1'-binaphthyl |
| CDCl₃ | Deuterochloroform |
| DC | Dünnschichtchromatographie |
| DCI | direkte chemische Ionisation (bei MS) |
| DCM | Dichlormethan |
| DIEA | *N*,*N*-Diisopropylethylamin |
| DMSO | Dimethylsulfoxid |
| DMF | *N*,*N*-Dimethylformamid |
| d. Th. | der Theorie |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| ggfs. | gegebenenfalls |
| H | Stunde |
| HPLC | Hochdruck-, Hochleistungsflüssigchromatographie |
| konz. | konzentriert |
| LC-MS | Flüssigchromatographie-gekoppelte Massenspektroskopie |
| LDA | Lithium-Diisopropylamid |
| Min | Minuten |
| MS | Massenspektroskopie |
| MTBE | Methyl-*tert*-butylether |
| NMR | Kernresonanzspektroskopie |
| Pd-C | Palladium auf Kohle |
| proz. | prozentig |
| RP-HPLC | Reverse Phase HPLC |
| RT | Raumtemperatur |
| Rₜ | Retentionszeit (bei HPLC) |
| THF | Tetrahydrofuran |

### Allgemeine Methoden LC-MS und HPLC:

**Methode 1 (analytische HPLC):** Instrument: HP 1100 mit DAD-Detektion; Säule: Kromasil RP-18, 60 mm x 2 mm, 3.5 µm; Eluent A: 5 ml Perchlorsäure/l Wasser, Eluent B: Acetonitril; Gradient: 0 min 2%B, 0.5 min 2%B, 4.5 min 90%B, 6.5 min 90%B; Fluss: 0.75 ml/min; Ofen: 30°C; UV-Detektion: 210 nm.
**Methode 2 (präparative HPLC, Labor-HPLC):** Säule: CromSil C18, 250 x 30 mm; Fluß: 50 ml/min; Laufzeit: 38 min; Eluent A: Wasser, Eluent B: Acetonitril, Gradient: 10%B (3 min) -> 90%B (31 min) -> 90%B (34 min) -> 10%B (34.01 min); UV-Detektion: 210 nm.
**Methode 3 (LCMS):** Instrument: Micromass Quattro LCZ, mit HPLC Agilent Serie 1100; Säule: Grom-SIL120 ODS-4 HE, 50 mm x 2.0 mm, 3 µm; Eluent A: 1 1 Wasser + 1 ml 50%ige Ameisensäure, Eluent B: 1 1 Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

### Ausgangsverbindungen

### Beispiel 1A

### Pyridin-4-yl-tert-butylcarbamat

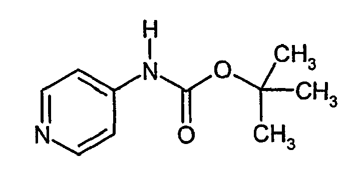

Zu einer Lösung von 5.1 g (23.4 mmol) Di-*tert*-butyldicarbonat in 20 ml THF werden portionsweise 2.0 g (21.3 mmol) 4-Aminopyridin gegeben. Nach vollständiger Zugabe wird 1h bei Raumtemperatur nachgerührt, anschließend das Lösungsmittel im Vakuum entfernt und der so erhaltene Rückstand in Diethylether suspendiert. Der Feststoff wird abfiltriert und im Vakuum getrocknet. Ausbeute: 3.43 g (83% d. Th.)
HPLC (Methode 1): Rₜ = 3.42 min
MS (ESI-pos): m/z = 195 [M+H]⁺

### Beispiel 2A

### Pyridin-3-yl-tert-butylcarbamat

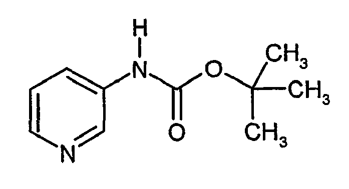

Zu einer Lösung von 5.0 g (40.9 mmol) Nicotinsäureamid in 100 ml *tert*-Butanol werden 18.2 g (40.9 mmol) Blei(IV)acetat gegeben und das Reaktionsgemisch 4h unter Rückfluss gerührt. Anschließend wird der Ansatz über Kieselgur filtriert, das Lösungsmittel im Vakuum entfernt und der Rückstand mit Diethylether aufgenommen. Die organische Phase wird mit gesättigter Natriumhydrogencarbonatlösung sowie gesättigter Natriumchloridlösung gewaschen, über Natriumsulfat getrocknet und filtriert. Die Lösung wird mit Pentan versetzt und der entstandene Niederschlag abfiltriert und getrocknet. Ausbeute: 2.79 g (35% d. Th.).
HPLC (Methode 1): Rₜ = 3.36 min
MS (ESI-pos): m/z = 195 [M+H]⁺

### Beispiel 3A

### (3-Formylpyridin-4-yl)-tert-butylcarbamat

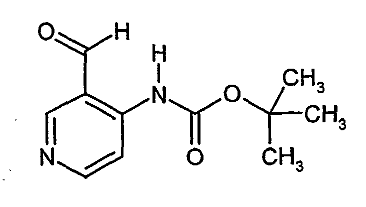

In 75 ml Tetrahydrofuran werden 4.12 g (21.3 mmol) des Boc-geschützten Aminopyridins aus Beispiel 1A gelöst, unter Argon die Lösung auf -78°C gekühlt und tropfenweise mit 34 ml einer 1.5 M Lösung (51.1 mmol) von *tert*-Butyllithium in Pentan versetzt. Die Zugabe erfolgt so, dass die Innentemperatur unter -65°C bleibt. Nach vollständiger Zugabe wird noch 1h bei -20°C nachgerührt. Danach wird der Ansatz mit 10.6 ml (138.3 mmol) absolutem *N*,*N*-Dimethylformamid versetzt, so daß bei der Zugabe die Reaktionstemperatur unter -15°C bleibt. Die Reaktion wird 16h bei Raumtemperatur nachgerührt, anschließend unter Eiskühlung mit 1N Salzsäure versetzt. Der pH-Wert wird mit festem Natriumcarbonat auf pH 7 eingestellt, der Ansatz mit Essigsäureethylester versetzt und die organische Phase mit Wasser sowie gesättigter Natriumchloridlösung gewaschen. Die organische Phase wird abgetrennt, über Natriumsulfat getrocknet und das Lösungsmittel im Vakuum entfernt. Das Produkt wird mittels Chromatographie an Kieselgel mit Cyclohexan/Essigsäureethylester (3:2 v/v) gereinigt. Man erhält 3.15 g (63% d. Th.) Produkt.
HPLC (Methode 1): Rₜ = 3.65 min
MS (ESI-pos): m/z = 223 [M+H]⁺

### Beispiel 4A

### (4-Formylpyridin-3-yl)-tert-butylcarbamat

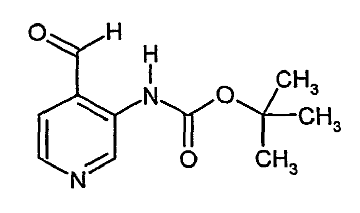

In 50 ml Tetrahydrofuran werden 2.7 g (13.9 mmol) des Boc-geschützten Aminopyridins aus Beispiel 2A gelöst, unter Argon die Lösung auf -78°C gekühlt und tropfenweise mit 22.4 ml einer 1.5 M Lösung (33.4 mmol) von *tert*-Butyllithium in Pentan versetzt. Die Zugabe erfolgt so, dass die Innentemperatur unter -65°C bleibt. Nach vollständiger Zugabe wird noch 1h bei -20°C nachgerührt. Danach wird der Ansatz mit 4.6 ml (41.7 mmol) *N*-Formlypiperidin versetzt, so daß bei der Zugabe die Reaktionstemperatur unter -15°C bleibt. Die Reaktion wird 16h bei Raumtemperatur nachgerührt, anschließend unter Eiskühlung mit 1N Salzsäure versetzt. Der pH wird mit festem Natriumcarbonat auf pH 7 eingestellt, der Ansatz mit Essigsäureethylester versetzt und die organische Phase mit Wasser sowie gesättigter Natriumchloridlösung gewaschen. Die organische Phase wird abgetrennt, über Natriumsulfat getrocknet und das Lösungsmittel im Vakuum entfernt. Das Produkt wird mittels Chromatographie an Kieselgel mit Cyclohexan/Essigsäureethylester (7:3 v/v) gereinigt. Man erhält 1.54 g (49% d. Th.) Produkt.
HPLC (Methode 1): Rₜ = 3.40 min
MS (ESI-pos): m/z = 223 [M+H]⁺

### Beispiel 5A

### 3-Amino-2-brompyridin

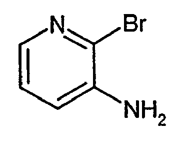

In 200 ml Acetonitril werden 4.00 g (42.5 mmol) 3-Aminopyridin gelöst, mit 8.32 g (46.8 mmol) *N*-Bromsuccinimid versetzt und unter Lichtausschluss 20h bei Raumtemperatur gerührt. Anschließend wird das Reaktionsgemisch eingeengt, in Essigsäureethylester suspendiert und mit gesättigter, wässriger Natriumhydrogencarbonatlösung und gesättigter, wässriger Natriumchloridlösung gewaschen. Die organische Phase wird über Natriumsulfat getrocknet, filtriert und eingeengt. An Kieselgel wird säulenchromatographisch mit Cyclohexan/ Essigsäureethylester (1:1) gereinigt. Man erhält 969 mg (12% d. Th.) Produkt.
HPLC (Methode 1): Rₜ = 1.08 min.
MS (ESI-pos): m/z = 173 [M+H]⁺

### Beispiel 6A

### (2E)-3-(3-Aminopyridin-2-yl)acrylsäure-tert-butylester

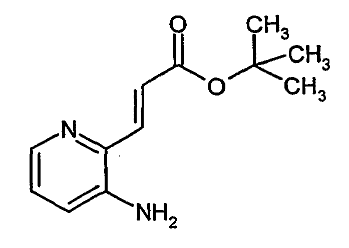

In 15 ml Acetonitril werden 950 mg (4.94 mmol) des Bromides aus Beispiel 5A, 1900 mg (14.83 mmol) Acrylsäure-*tert*-butylester, 330 mg (1.50 mmol) Palladium(II)acetat, 450 mg (1.50 mmol) Tri-ortho-tolylphospin gelöst und 1000 mg (9.88 mmol) Triethylamin zugesetzt. Man rührt 16h unter Rückfluss. Das Reaktionsgemisch wird eingeengt, mit Essigsäureethylester versetzt und mit gesättigter, wässriger Natriumchloridlösung gewaschen. Die organische Phase wird über Natriumsulfat getrocknet, filtriert, eingeengt und säulenchromatographisch an Kieselgel mit Cyclohexan/Essigsäureethylester (7:3) gereinigt. Man erhält 95 mg (6% d. Th.) Produkt.

### Beispiel 7A

### (2E)-3-{4-[(tert-Butoxycarbonyl)amino]pyridin-3-yl}acrylsäuremethylester

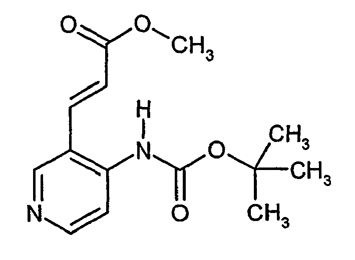

Eine Suspension von 3.0 g (13.5 mmol) des Aldehyds aus Beispiel 3A, 3.12 g (14.8 mmol) Methyldiethylphosphonoacetat und 623 mg (14.8 mmol) Lithiumhydroxydmonohydrat in 30 ml Tetrahydrofuran wird 16h bei Raumtemperatur gerührt. Anschließend wird mit 30 ml Wasser versetzt und mit Essigsäureethylester extrahiert. Die organische Phase wird abgetrennt, mit gesättigter Natriumchloridlösung gewaschen, über Natriumsulfat getrocknet, filtriert und das Lösungsmittel im Vakuum entfernt. Der Rückstand wird bei 100°C / 1 mbar getrocknet. Man erhält 3.5 g (89% d. Th.) der Titelverbindung.
HPLC (Methode 1): Rₜ = 3.71 min
MS (ESI-pos): m/z = 279 [M+H]⁺

### Beispiel 8A

### (2E)-3-{3-[(tert-Butoxycarbonyl)amino]pyridin-4-yl}acrylsäuremethylester

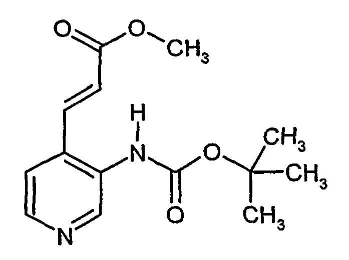

Eine Suspension von 1.48 g (6.66 mmol) des Aldehyds aus Beispiel 4A, 1.54 g (7.33 mmol) Methyldiethylphosphonoacetat und 307 mg (7.33 mmol) Lithiumhydroxydmonohydrat in 15 ml Tetrahydrofuran wird 16h bei Raumtemperatur gerührt. Anschließend wird mit 15 ml Wasser versetzt und mit Essigsäureethylester extrahiert. Die organische Phase wird abgetrennt, mit gesättigter Natriumchloridlösung gewaschen, über Natriumsulfat getrocknet, filtriert und das Lösungsmittel im Vakuum entfernt. Der Rückstand wird bei 75°C / 1 mbar getrocknet. Man erhält 1.82 g (98% d. Th.) der Titelverbindung.
HPLC (Methode 1): Rₜ = 3.73 min
MS (ESI-pos): m/z = 279 [M+H]⁺

### Beispiel 9A

### (2E)-3-{4-Aminopyridin-3-yl}acrylsäuremethylester

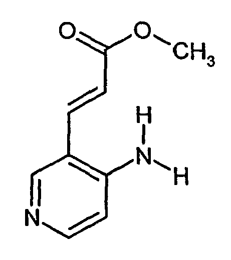

Bei 0°C werden 900 mg (3.23 mmol) des Boc-geschützten Aminopyridins aus Beispiel 7A in 3 ml Trifluoressigsäure gelöst. Anschließend wird das Reaktionsgemisch 1h bei Raumtemperatur gerührt, der Ansatz in eiskalte Natriumhydrogencarbonatlösung eingetragen, 30 min gerührt und der entstandene Niederschlag abfiltriert, mit Wasser gewaschen und getrocknet. Man erhält 242 mg (42% d. Th.) der Titelverbindung.

Aus der Mutterlauge können durch Einstellen des pH-Wertes auf pH 10 und Extraktion mit Essigsäureethylester weitere 105 mg (18% d. Th.) Produkt als Öl isoliert werden. Beide Fraktionen unterscheiden sich vermutlich durch ihren Protonierungsgrad.
HPLC (Methode 1): Rₜ = 2.67 min
MS (CI-pos): m/z = 179 [M+H]⁺

### Beispiel 10A

### (2E)-3-{3-Aminopyridin-4-yl}acrylsäuremethylester

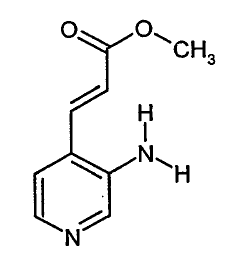

Bei Raumtemperatur werden 1.8 g (6.47 mmol) des Boc-geschützten Aminopyridins aus Beispiel 8A in 18 ml Methanol gelöst und die Lösung mit 1 ml konzentrierter Salzsäure versetzt. Anschließend wird das Reaktionsgemisch 4h bei 70°C gerührt, der Ansatz in 50 ml Natriumhydrogencarbonatlösung eingetragen, der pH-Wert durch Zugabe von 20%iger Natronlauge auf pH 14 eingestellt und mit Essigsäureethylester extrahiert. Die organische Phase wird mit konzentrierter Natriumchloridlösung gewaschen, über Natriumsulfat getrocknet und im Vakuum das Lösungsmittel entfernt. Ausbeute: 935 mg (79% d. Th.).
HPLC (Methode 1): Rₜ = 2.88 min
MS (ESI-pos): m/z = 179 [M+H]⁺

### Allgemeine Arbeitsvorschrift [A]: Darstellung von Iminonhosphoranen aus Aminopyridinen

In Acetonitril werden 1.0 eq Aminopyridin, 2.0 eq. Triphenylphosphin, 10.0 eq. Tetrachlorkohlenstoff sowie 10.0 eq. Triethylamin suspendiert (ca. 0.33 M bezogen auf das Aminopyridin). Das Reaktionsgemisch wird 16h bei Raumtemperatur gerührt und das Lösungsmittel im Vakuum entfernt. Das Rohprodukt wird ohne weitere Aufreinigung umgesetzt oder säulenchromatographisch an Kieselgel gereinigt.

### Beispiel 11A

### (2E)-3-{3-[(Triphenylphosphoranyliden)amino]pyridin-2-yl}acrylsäure-tert-butylester

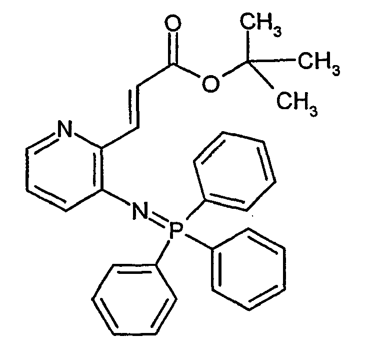

Ausgehend von 95 mg (0.28 mmol) Aminopyridin aus Beispiel 6A wird gemäß allgemeiner Arbeitsvorschrift [A] das Rohprodukt erhalten, das säulenchromatographisch an Kieselgel mit Cyclohexan/Essigsäureethylester (7:3) gereinigt wird. Man erhält 70 mg (51% d. Th.) Produkt.
HPLC (Methode 1): Rₜ = 4.83 min.
MS (ESI-pos): m/z = 481 [M+H]⁺

### Beispiel 12A

### (2E)-3-{4-[(Triphenylphosphoranyliden)amino]pyridin-3-yl}acrylsäuremethylester

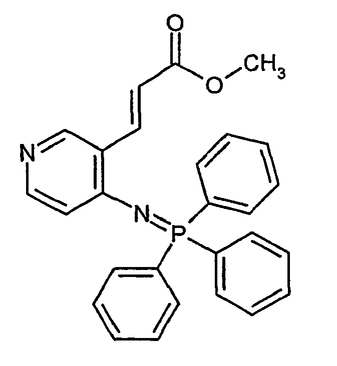

Ausgehend von 320 mg (1.8 mmol) Aminopyridin aus Beispiel 9A werden gemäß allgemeiner Arbeitsvorschrift [A] 1650 mg Rohprodukt erhalten, das ohne weitere Reinigung umgesetzt wird.

### Beispiel 13A

### (2E)-3-{3-[(Triphenylphosphoranyliden)amino]pyridin-4-yl}acrylsäuremethylester

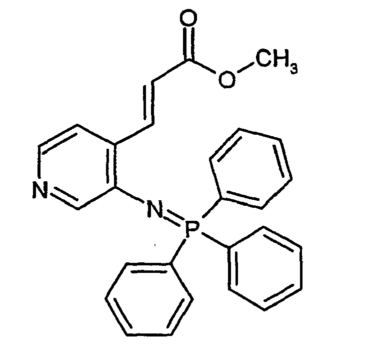

Ausgehend von 900 mg (4.89 mmol) Aminopyridin aus Beispiel 10A werden gemäß allgemeiner Arbeitsvorschrift [A] 1910 mg Rohprodukt erhalten, das ohne weitere Reinigung umgesetzt wird.
MS (ESI-pos): m/z = 439 [M+H]⁺

### Allgemeine Arbeitsvorschrift [B]: Darstellung von Carbodiimiden aus Iminophosphoranen durch Umsetzung mit Isocyanaten

In Dichlormethan werden 1.0 Äq. Iminophosphoran (ggf. als Rohprodukt) gelöst, mit 1.1 Äq. Isocyanat versetzt und das Reaktionsgemisch 16h bei Raumtemperatur gerührt. Das so erhaltene Rohprodukt wird direkt weiter umgesetzt.

### Beispiel 14A

### (2E)-3-{3-[({[3-(Trifluormethyl)phenyl]imino}methylen)amino]pyridin-2-yl}acrylsäure-tert-butylester

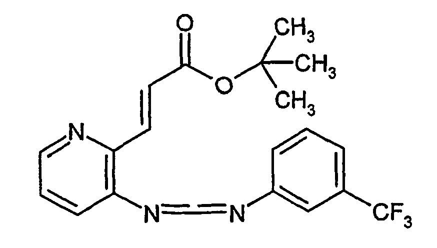

65 mg (0.14 mmol) Iminophosphoran aus Beispiel 11A werden gemäß allgemeiner Arbeitsvorschrift [B] mit 27 mg (0.14 mmol) 3-Trifluormethylphenylisocyanat umgesetzt und das Rohprodukt ohne Reinigung weiter umgesetzt.

### Beispiel 15A

### (2E)-3-{4-[({[3-(Trifluormethyl)phenyl]imino}methylen)amino]pyridin-3-yl}acrylsäuremethylester

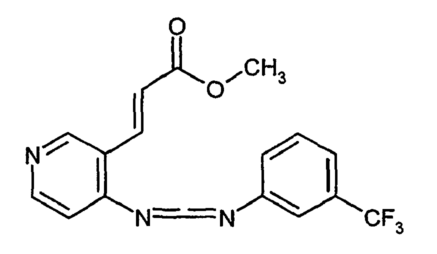

300 mg (0.34 mmol) Iminophosphoran aus Beispiel 12A werden gemäß allgemeiner Arbeitsvorschrift [B] mit 70 mg (0.38 mmol) 3-Trifluormethylphenylisocyanat umgesetzt und das Rohprodukt ohne Reinigung weiter umgesetzt.

### Beispiel 16A

### (2E)-3-[4-({[(2-Methoxy-5-methylphenyl)imino]methylen}amino)pyridin-3-yl]acrylsäuremethylester

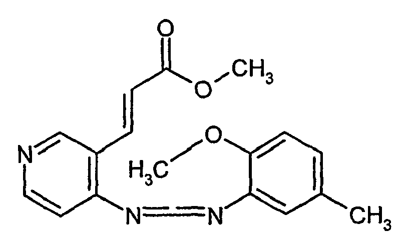

150 mg (0.28 mmol) Iminophosphoran aus Beispiel 12A werden gemäß allgemeiner Arbeitsvorschrift [B] mit 67 mg (0.42 mmol) 1-Methoxy-4-methylphenylisocyanat umgesetzt und das Rohprodukt ohne Reinigung weiter umgesetzt.

### Beispiel 17A

### (2E)-3-{3-[({[3-(Trifluormethyl)phenyl]imino}methylen)amino]pyridin-4-yl}acrylsäuremethylester

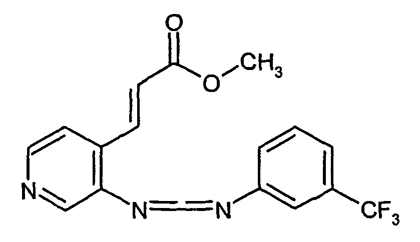

1000 mg (1.37 mmol) Aminopyridin aus Beispiel 13A werden gemäß allgemeiner Arbeitsvorschrift [B] mit 282 mg (1.51 mmol) 3-Trifluormethylphenylisocyanat umgesetzt und das Rohprodukt ohne Reinigung weiter umgesetzt.

### Beispiel 18A

### (2E)-3-{4-[({[2-Methoxy-5-(trifluormethyl)phenyl]imino}methylen)amino]pyridin-3-yl}acrylsäuremethylester

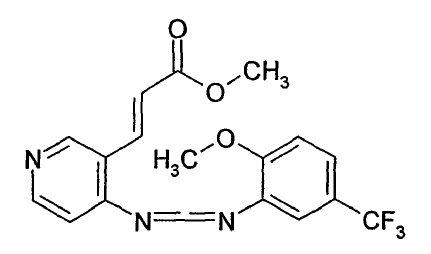

1017 mg (1.90 mmol) Iminophosphoran aus Beispiel 12A werden gemäß allgemeiner Arbeitsvorschrift [B] mit 413 mg (1.90 mmol) 1-Methoxy-4-trifluormethylphenylisocyanat umgesetzt und das Rohprodukt ohne Reinigung weiter umgesetzt.

### Beispiel 19A

### 2-Isocyanato-1-methoxy-4-(trifluormethyl)benzol

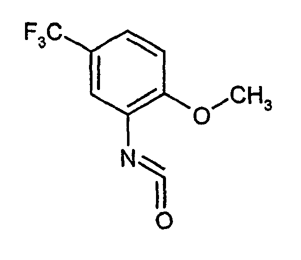

In 100 ml Dichlormethan werden 3 g (15.69 mmol) 2-Methoxy-5-trifluormethylanilin gelöst und mit 6.73 g (31.39 mmol) 1,8-Bis(dimethylamino)naphthalin versetzt. Bei 0-5°C werden 2.24 g (11.3 mmol) Chlorameisensäuretrichlormethylester, gelöst in 50 ml Dichlormethan, zugetropft und 30 min bei 0°C sowie 60 min bei Raumtemperatur gerührt. Bei 0°C wird mit 1N Salzsäure, Eiswasser und Natriumhydrogencarbonatlösung gewaschen. Nach Trocknen über Magnesiumsulfat und Abdestillieren des Lösungsmittels wird das Produkt erhalten. Das Isocyanat wird anschließend ohne weitere Reinigung in den folgenden Reaktionen umgesetzt. Ausbeute: 3 g (88% d. Th.)

### Allgemeine Arbeitsvorschrift [C]: Umsetzung von Carbodiimiden mit Phenylpiperazinen zu Dihydropyridopyrimidinylessigsäureestern

In Dichlormethan werden 1.0 eq Carbodiimid (ggfs. als Rohprodukt) gelöst, mit 1.05 eq Phenylpiperazin sowie einer Spatelspitze Kieselgel versetzt und das Reaktionsgemisch 16h unter Rückfluss gerührt. Anschließend wird das Lösungsmitztel in Vakuum entfernt und das Produkt mittels Chromatographie an Kieselgel oder mittels präparativer HPLC (Methode 2) gereinigt.

### Beispiel 20A

### {2-[4-(4-Fluorphenyl)piperazin-1-yl]-3-[3-(trifluormethyl)phenyl]-3,4-dihydropyrido[3,4-d]pyrimidin-4-yl}essigsäuremethylester

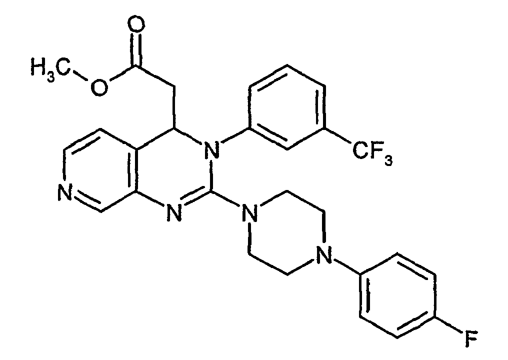

Ausgehend von 250 mg (0.34 mmol) des Carbodiimids aus Beispiel 17A und 65 mg (0.36 mmol) 4-Fluorphenylpiperazin werden gemäß allgemeiner Arbeitsvorschrift [C] und zweifacher Reinigung mittels präparativer HPLC 64 mg (33% d. Th.) Produkt erhalten.
HPLC (Methode 1): Rₜ = 4.42 min
MS (ESI-pos): m/z = 528 [M+H]⁺

### Beispiel 21A

### {2-[4-(3-Methylphenyl)piperazin-1-yl]-3-[3-(trifluormethyl)phenyl]-3,4-dihydropyrido[3,4-d]pyrimidin-4-yl}essigsäuremethylester

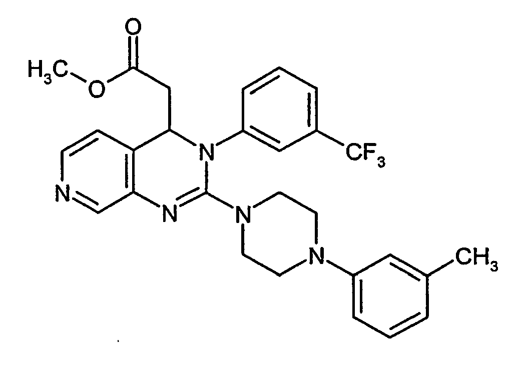

Ausgehend von 250 mg (0.34 mmol) des Carbodiimids aus Beispiel 17A und 63 mg (0.36 mmol) 3-Methylphenylpiperazin werden gemäß allgemeiner Arbeitsvorschrift [C] und Reinigung mittels präparativer HPLC 88 mg (48% d. Th.) Produkt erhalten.
HPLC (Methode 1): Rₜ = 4.42 min
MS (ESI-pos): m/z = 524 [M+H]⁺

### Beispiel 22A

### {2-[4-(3-Chlorphenyl)piperazin-1-yl]-3-[3-(trifluormethyl)phenyl]-3,4-dihydropyrido[3,4-d]pyrimidin-4-yl}essigsäuremethylester

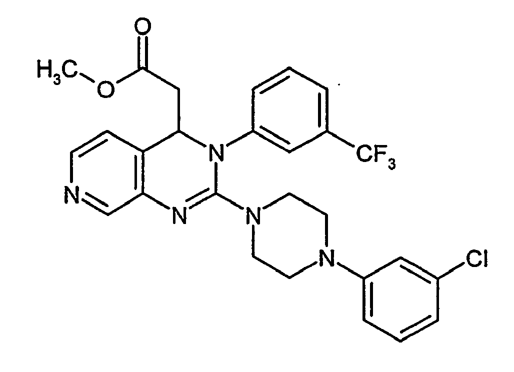

Ausgehend von 250 mg (0.34 mmol) des Carbodiimids aus Beispiel 17A und 71 mg (0.36 mmol) 3-Chlorphenylpiperazin werden gemäß allgemeiner Arbeitsvorschrift [C] und Reinigung mittels präparativer HPLC 102 mg (53% d. Th.) Produkt erhalten.
HPLC (Methode 1): Rₜ = 4.65 min
MS (ESI-pos): m/z = 544 [M+H]⁺

### Beispiel 23A

### {2-[4-(3-Methoxyphenyl)piperazin-1-yl]-3-[3-(trifluormethyl)phenyl]-3,4-dihydropyrido[3,4-d]pyrimidin-4-yl}essigsäuremethylester

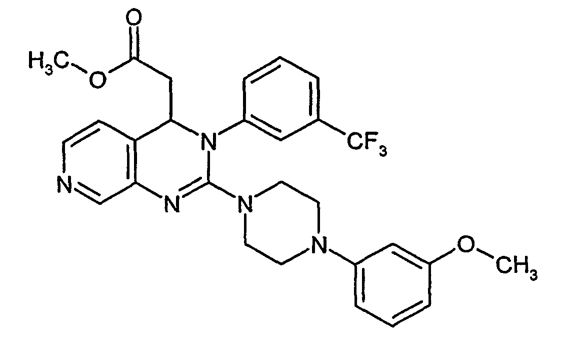

Ausgehend von 250 mg (0.34 mmol) des Carbodiimids aus Beispiel 17A und 69 mg (0.36 mmol) 3-Methoxyphenylpiperazin werden gemäß allgemeiner Arbeitsvorschrift [C] und Reinigung mittels präparativer HPLC 90 mg (46% d. Th.) Produkt erhalten.
HPLC (Methode 1): Rₜ = 4.40 min
MS (ESI-pos): m/z = 540 [M+H]⁺

### Beispiel 24A

### {2-[4-(4-Fluorphenyl)piperazin-1-yl]-3-[3-(trifluormethyl)phenyl]-3,4-dihydropyrido[4,3-d]pyrimidin-4-yl}essigsäuremethylester

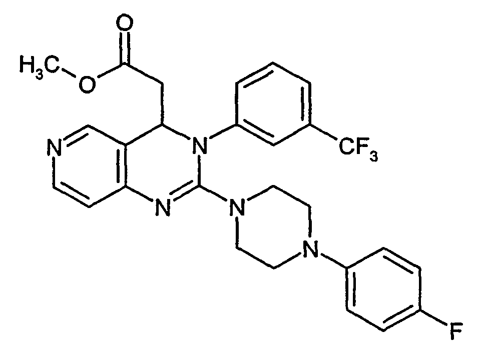

Ausgehend von 107 mg (0.31 mmol) des Carbodiimids aus Beispiel 15A und 56 mg (0.31 mmol) 4-Fluorphenylpiperazin werden gemäß allgemeiner Arbeitsvorschrift [C] und Reinigung mittels präparativer HPLC 50 mg (28% d. Th.) Produkt erhalten.
HPLC (Methode 1): Rₜ = 4.60 min
MS (ESI-pos): m/z = 528 [M+H]⁺

### Beispiel 25A

### {2-[4-(3-Methoxyphenyl)piperazin-1-yl]-3-[3-(trifluormethyl)phenyl]-3,4-dihydropyrido[4,3-d]pyrimidin-4-yl}essigsäuremethylester

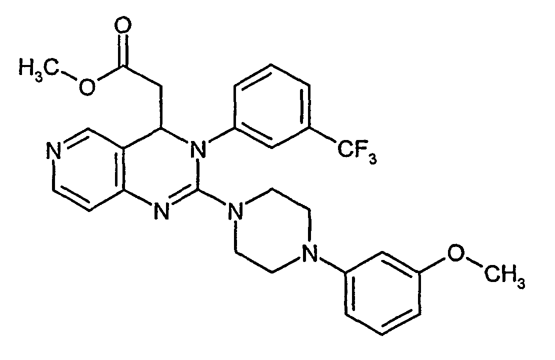

Ausgehend von 107 mg (0.31 mmol) des Carbodiimids aus Beispiel 15A und 59 mg (0.31 mmol) 3-Methoxyphenylpiperazin werden gemäß allgemeiner Arbeitsvorschrift [C] und Reinigung mittels präparativer HPLC 40 mg (23% d. Th.) Produkt erhalten.
HPLC (Methode 1): Rₜ = 4.60 min
MS (ESI-pos): m/z = 540 [M+H]⁺

### Beispiel 26A

### {2-[4-(4-Fluorphenyl)piperazin-1-yl]-3-[3-(trifluormethyl)phenyl]-3,4-dihydropyrido[3,2-d]pyrimidin-4-yl}essigsäure-tert-butylester

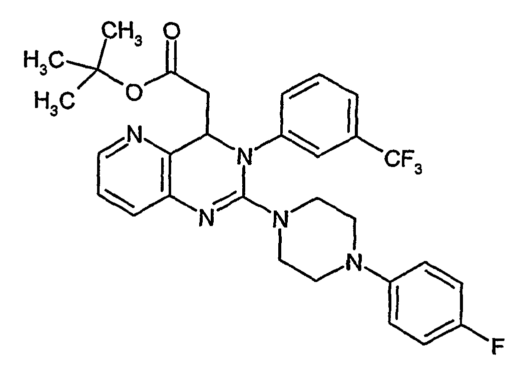

Ausgehend von 53 mg (0.14 mmol) des Carbodiimids aus Beispiel 14A und 24 mg (0.14 mmol) 4-Fluorphenylpiperazin werden gemäß allgemeiner Arbeitsvorschrift [C] und Reinigung an präparativer HPLC 38 mg (48% d. Th.) Produkt erhalten.
HPLC (Methode 1): Rₜ = 4.87 min
MS (ESI-pos): m/z = 570 [M+H]⁺

### Beispiel 27A

### [2-[4-(4-Fluorphenyl)piperazin-1-yl]-3-(2-methoxy-5-methylphenyl)-3,4-dihydropyrido[4,3-d]pyrimidin-4-yl]essigsäuremethylester

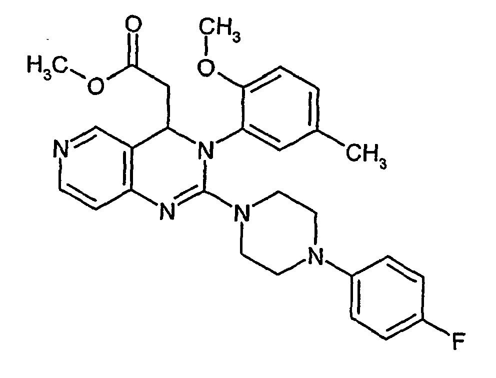

Ausgehend von 91 mg (0.28 mmol) des Carbodiimids aus Beispiel 16A und 76 mg (0.42 mmol) 4-Fluorphenylpiperazin werden gemäß allgemeiner Arbeitsvorschrift [C] und Reinigung mittels präparativer HPLC 14 mg (9% d. Th.) Produkt erhalten.
HPLC (Methode 1): Rₜ = 4.23 min
MS (ESI-pos): m/z = 504 [M+H]⁺

### Beispiel 28A

### {2-[4-(4-Fluorphenyl)piperazin-1-yl]-3-[2-methoxy-5-(trifluormethyl)phenyl]-3,4-dihydro-pyrido[4,3-d]pyrimidin-4-yl}essigsäuremethylester

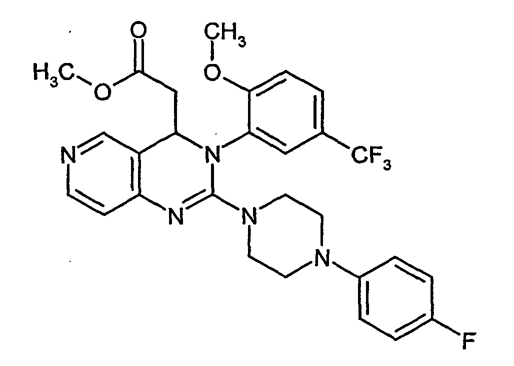

Ausgehend von 565 mg (1.50 mmol) des Carbodiimids aus Beispiel 18A und 297 mg (1.65 mmol) 4-Fluorphenylpiperazin werden gemäß allgemeiner Arbeitsvorschrift [C] und Reinigung mittels präparativer HPLC 90 mg (10% d. Th.) Produkt erhalten.
HPLC (Methode 1): Rₜ = 4.44 min
MS (ESI-pos): m/z = 558 [M+H]⁺

### Ausführungsbeispiele

### Allgemeine Arbeitsvorschrift [D]: Esterverseifung

In Dioxan werden 1.0 Äquivalente des Esters gelöst (ca. 0.5 M Lösung), dann 3.0 Äquivalente 1N Natronlauge zugegeben und das Reaktionsgemisch über 16h bei 50°C gerührt. Anschließend wird der Ansatz mit 1N Salzsäure auf pH 5 eingestellt, das Lösungsmittel im Vakuum entfernt und das Produkt mittels Chromatographie an Kieselgel oder präparativer HPLC gereinigt.

### Beispiel 1

### {2-[4-(4-Fluorphenyl)piperazin-1-yl]-3-[3-(trifluormethyl)phenyl]-3,4-dihydropyrido[3,4-d]pyrimidin-4-yl}essigsäure Hydrochlorid

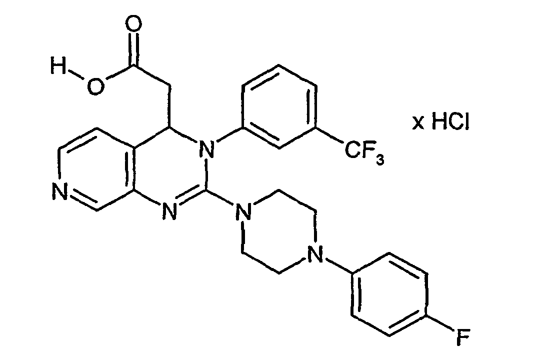

Ausgehend von 44 mg (0.08 mmol) des Esters aus Beispiel 20A werden nach Umsetzung gemäß allgemeiner Arbeitsvorschrift [D] und Reinigung mittels präparativer HPLC 24 mg (52% d. Th.) Produkt erhalten.
HPLC (Methode 1): Rₜ = 4.19 min
MS (ESI-pos): m/z = 514 [M+H-HCl]⁺
¹H-NMR (400 MHz, CD₃CN+DMSO-d₆): δ [ppm] = 8.34 (s, 1H); 8.11 (d, 1H); 7.62 (s, 1H); 7.47 (t, 1H); 7.39-7.37 (m, 2H); 7.07 (t, 1H); 6.99 (t, 1H); 6.91-6.87 (m, 1H); 5.27-5.23 (m, 1H); 3.58-3.53 (m, 2H), weitere Protonen unter dem Lösungsmittel- bzw. Wassersignal.

### Beispiel 2

### {2-[4-(3-Methylphenyl)piperazin-1-yl]-3-[3-(trifluormethyl)phenyl]-3,4-dihydropyrido[3,4-d]pyrimidin-4-yl}essigsäure Hydrochlorid

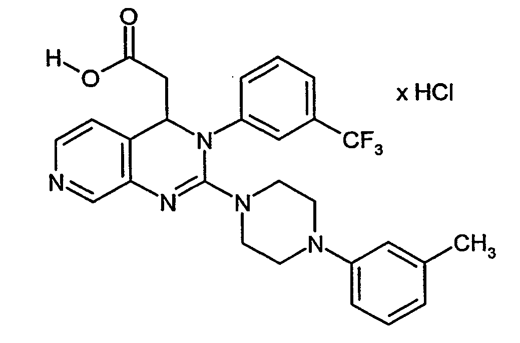

Ausgehend von 70 mg (0.13 mmol) des Esters aus Beispiel 21A werden nach Umsetzung gemäß allgemeiner Arbeitsvorschrift [D] und Reinigung mittels präparativer HPLC 51 mg (71% d. Th.) Produkt erhalten.
HPLC (Methode 1): Rₜ = 4.21 min
MS (ESI-pos): m/z = 510 [M+H-HCl]⁺
¹H-NMR (400 MHz, CD₃CN+DMSO-d₆): δ [ppm] = 8.34 (s, 1H); 8.12 (d, 1H); 7.62 (s, 1H); 7.44 (t, 1H); 7.38-7.36 (m, 2H); 7.10 (t, 1H); 7.05 (d, 1H); 6.89-6.88 (m, 1H); 6.72-6.64 (m, 3H); 5.26-5.22 (m, 1H); 3.59-3.53 (m, 2H); 3.08-2.95 (m, 3H); 2.72-2.65 (m, 1H); 2.48-2.46 (m, 1H); 2.25 (s, 3H).

### Beispiel 3

### {2-[4-(3-Chlorphenyl)piperazin-1-yl]-3-[3-(trifluormethyl)phenyl]-3,4-dihydropyrido[3,4-d]pyrimidin-4-yl}essigsäure Hydrochlorid

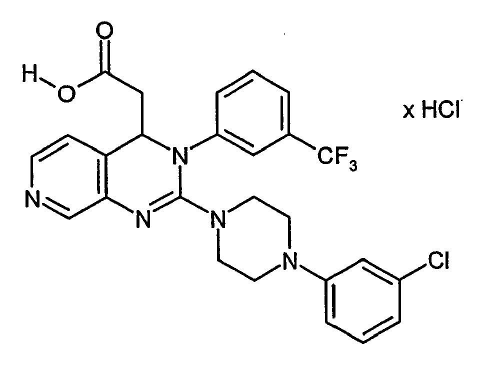

Ausgehend von 84 mg (0.15 mmol) des Esters aus Beispiel 22A werden nach Umsetzung gemäß allgemeiner Arbeitsvorschrift [D] und Reinigung mittels präparativer HPLC 44 mg (52% d. Th.) Produkt erhalten.
HPLC (Methode 1): Rₜ = 4.43 min.
MS (ESI-pos): m/z = 530 [M+H-HCl]⁺
¹H-NMR (400 MHz, CD₃CN+DMSO-d₆): δ [ppm] = 8.34 (s, 1H); 8.12 (d, 1H); 7.62 (s, 1H); 7.46 (t, 1H); 7.39-7.35 (m, 2H); 7.19 (t, 1H); 7.05 (d, 1H); 6.89-6.88 (m, 1H); 6.83-6.79 (m, 2H); 5.26-5.22 (m, 1H); 3.59-3.53 (m, 2H); 3.13-3.02 (m, 3H); 2.72-2.65 (m, 1H); 2.48-2.41 (m, 1H).

### Beispiel 4

### {2-[4-(3-Methoxyphenyl)piperazin-1yl]-3-[3-(trifluormethyl)phenyl]-3,4-dihydropyrido[3,4-d]pyrimidin-4-yl}essigsäure Hydrochlorid

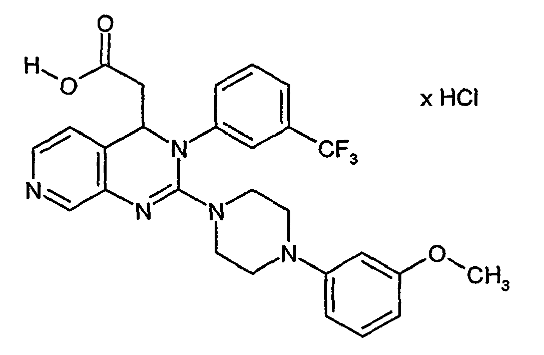

Ausgehend von 72 mg (0.13 mmol) des Esters aus Beispiel 23A werden nach Umsetzung gemäß allgemeiner Arbeitsvorschrift [D] und Reinigung mittels präparativer HPLC 48 mg (67% d. Th.) Produkt erhalten.
HPLC (Methode 1): Rₜ = 4.18 min.
MS (ESI-pos): m/z = 526 [M+H-HCl]⁺
¹H-NMR (400 MHz, CD₃CN+DMSO-d₆): δ [ppm] = 8.35 (s, 1H); 8.12 (d, 1H); 7.56 (s, 1H); 7.46 (t, 1H); 7.39-7.36 (m, 2H); 7.12 (t, 1H); 7.05 (d, 1H); 6.50-6.47 (m, 1H); 6.43-6.39 (m, 2H); 5.26-5.22 (m, 1H); 3.73 (s, 3H); 3.59-3.52 (m, 2H); 3.09-2.98 (m, 3H); 2.72-2.65 (m, 1H); 2.48-2.46 (m, 1H).

### Beispiel 5

### {2-[4-(4-Fluorphenyl)piperazin-1-yl]-3-[3-(trifluormethyl)phenyl]-3,4-dihydropyrido[4,3-d]pyrimidin-4-yl}essigsäure Hydrochlorid

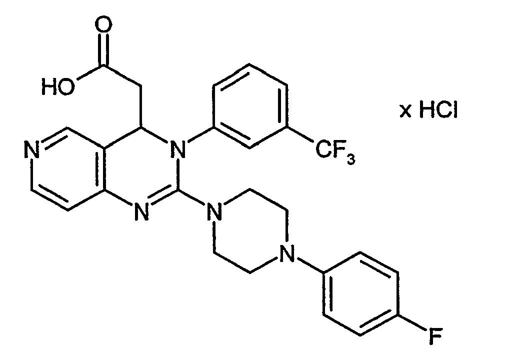

Ausgehend von 38 mg (0.07 mmol) des Esters aus Beispiel 24A werden nach Umsetzung gemäß allgemeiner Arbeitsvorschrift [D] und Reinigung mittels präparativer HPLC 24 mg (67% d. Th.) Produkt erhalten.
HPLC (Methode 1): Rₜ = 4.24 min.
MS (ESI-pos): m/z = 514 [M+H-HCl]⁺
¹H-NMR (400 MHz, CD₃CN): δ [ppm]= 8.22 (d, 1H); 8.18 (s, 1H); 7.45 (s, 1H); 7.40-7.36 (m, 3H); 7.03-6.83 (m, 5H); 5.28 (t, 1H); 3.603-3.50 (m, 4H); 2.99-2.90 (m, 4H); 2.78 (dd, 1H); 2.57 (dd, 1H).

### Beispiel 6

### {2-[4-(3-Methoxyphenyl)piperazin-1-yl]-3-[3-(trifluormethyl)phenyl]-3,4-dihydropyrido[4,3-d]pyrimidin-4-yl}essigsäure

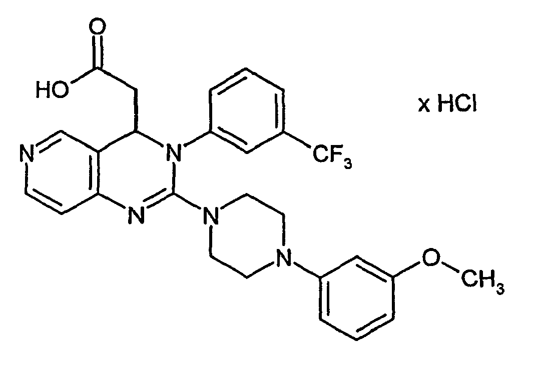

Ausgehend von 27 mg (0.05 mmol) des Esters aus Beispiel 25A werden nach Umsetzung gemäß allgemeiner Arbeitsvorschrift [D] und Reinigung mittels präparativer HPLC 19 mg (72% d. Th.) Produkt erhalten.
HPLC (Methode 1): Rₜ = 4.24 min.
MS (ESI-pos): m/z = 526 [M+H-HCl]⁺
¹H-NMR (400 MHz, CD₃CN): δ [ppm] = 8.24 (d, 1H); 8.18 (s, 1H); 7.45 (s, 1H); 7.40-7.35 (m, 3H); 7.12 (t, 1H); 7.01 (d, 1H); 6.45 (d, 1H); 6.41-6.38 (m, 2H); 5.28 (t, 1H); 3.72 (s, 3H); 3.60-3.54 (m, 4H); 3.07-2.78 (m, 4H); 2.55 (dd, 2H).

### Beispiel 7

### {2-[4-(4-Fluorphenyl)piperazin-1-yl]-3-[3-(trifluormethyl)phenyl]-3,4-dihydropyrido[3,2-d]pyrimidin-4-yl}essigsäure Hydrochlorid

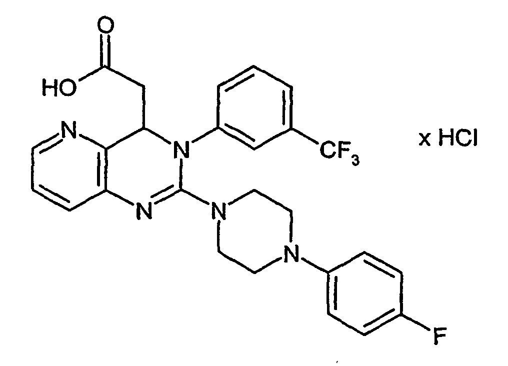

Ausgehend von 25 mg (0.04 mmol) des Esters aus Beispiel 26A werden nach Umsetzung gemäß allgemeiner Arbeitsvorschrift [D] und Reinigung mittels präparativer HPLC 16 mg (66% d. Th.) Produkt erhalten.
HPLC (Methode 1): Rₜ = 4.30 min.
MS (ESI-pos): m/z = 514 [M+H-HCl]⁺
¹H-NMR (400 MHz, CD₃CN): δ [ppm] = 8.12 (d, 1H); 7.60 (s, 1H); 7.45-7.39 (m, 4H); 7.22 (dd, 1H); 6.96 (t, 2H); 6.89-6.86 (m, 2H); 5.18 (dd, 1H); 3.68-3.59 (m, 4H); 3.06-2.99 (m, 4H); 2.76 (dd, 1H); 2.63 (dd, 1H).

### Beispiel 8

### [2-[4-(4-Fluorphenyl)piperazin-1-yl]-3-(2-methoxy-5-methylphenyl)-3,4-dihydropyrido[4,3-d]pyrimidin-4-yl]essigsäure

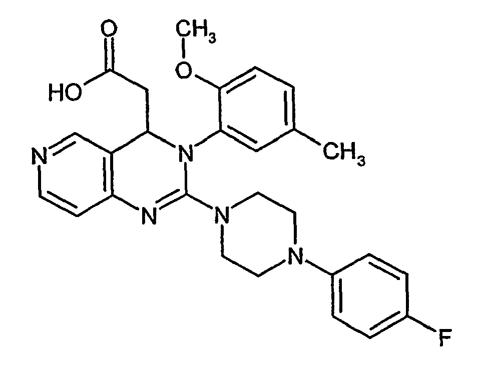

Ausgehend von 12 mg (0.02 mmol) des Esters aus Beispiel 27A werden nach Umsetzung gemäß allgemeiner Arbeitsvorschrift [D] und Reinigung mittels präparativer HPLC 11 mg (97% d. Th.) Produkt erhalten.
HPLC (Methode 1): Rₜ = 4.05 min.
MS (ESI-pos): m/z = 490 [M+H]⁺
¹H-NMR (400 MHz, CD₃CN): δ [ppm] = 8.14-8.10 (m, 2H); 7.15-6.81 (m, 8H); 4.86-4.84 (m, 1H); 3.77-3.73 (m, 4H); 3.50 (s, 3H); 2.96-2.92 (m, 2H); 2.78-2.74 (m, 2H); 2.49 (dd, 1H); 2.50-2.48 (m, 1H, teilweise unter CH₃-Signal); 2.44 (s, 3H).

### Beispiel 9

### {2-[4-(4-Fluorphenyl)piperazin-1-yl]-3-[2-methoxy-5-(trifluormethyl)phenyl]-3,4-dihydro-pyrido[4,3-d]pyrimidin-4-yl}essigsäure

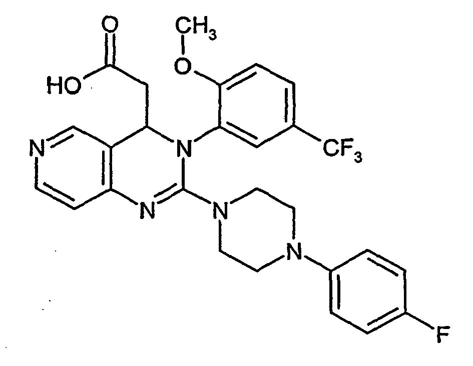

Ausgehend von 80 mg (0.14 mmol) des Esters aus Beispiel 28A werden nach Umsetzung gemäß allgemeiner Arbeitsvorschrift [D] und Reinigung mittels präparativer HPLC 27 mg (35% d. Th.) Produkt erhalten.
HPLC (Methode 3): Rₜ = 2.31 min.
MS (ESI-pos): m/z = 543 [M+H]⁺
¹H-NMR (400 MHz, CD₃CN): δ [ppm] = 8.26-8.20 (m, 2H); 7.55 (d, 1H); 7.16 (d, 1H); 7.10-6.78 (m, 6H); 5.00 (dd, 1H); 3.88-3.80 (m, 4H); 3.54 (s, 3H); 3.03 (dd, 1H); 2.96-2.91 (m, 2H); 2.79-2.77 (m, 2H); 2.55 (dd, 1H).

### B. Bewertung der physiologischen Wirksamkeit

Die *in vitro*-Wirkung der erfindungsgemäßen Verbindungen kann in folgenden Assays gezeigt werden:

### Anti-HCMV- (Anti-Humanes Cytomegalo-Virus) Zytopathogenitätstests

Die Testverbindungen werden als 50 millimolare (mM) Lösungen in Dimethysulfoxid (DMSO) eingesetzt. Ganciclovir^{®}, Foscarnet^{®} und Cidofovir^{®} dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 10⁴ Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5% CO₂ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque Multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50% im Vergleich zur unbehandelten Viruskontrolle hemmt;
SI (Selektivitätsindex) = CC₅₀ (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel Nr.** | **NHDF CC₅₀ [**µ**M]** | **HCMV EC₅₀ [**µ**M]** | **SI HCMV** |
|---|---|---|---|
| **4** | 94 | 0.2 | 470 |
| **8** | 250 | 0.14 | 1786 |
| **9** | 94 | 0.05 | 1880 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

### Tiere:

3-4 Wochen alte weibliche immundefiziente Mäuse (16-18 g), Fox Chase SCID oder Fox Chase SCID-NOD oder SCID-beige werden von kommerziellen Züchtern (Taconic M+B, Jackson, USA) bezogen. Die Tiere werden unter sterilen Bedingungen (einschließlich Streu und Futter) in Isolatoren gehalten.

### Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0.01-0.03 werden die virusinfizierten Zellen 5-10 Tage später geerntet und in Gegenwart von Minimal Essential Medium (MEM), 10% foetalem Kälberserum (FKS) mit 10% DMSO bei -40°C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot.

### Vorbereitung der Schwämme, Transplantation, Behandlung und Auswertung:

1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM + 10% FKS aufbewahrt. 1 x 10⁶ virusinfizierte NHDF-Zellen (Infektion mit HCMV-Davis oder HCMV AD169 M.O.I = 0.03) werden 3 Stunden nach Infektion abgelöst und in 20 µl MEM, 10% FKS auf einen feuchten Schwamm getropft. Ca. 16 Stunden später werden die mit den infizierten Zellen beladenen Schwämme mit 25 µl PBS / 0.1% BSA / 1 mM DTT mit 5 ng/µl basic Fibroblast Growth Factor (bFGF) inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber verschlossen. 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (14 Uhr) peroral mit Substanz behandelt. Die Tagesdosis beträgt beispielsweise 3 oder 10 oder 30 oder 60 oder 100 mg/kg Körpergewicht, das Applikationsvolumen 10 ml/kg Körpergewicht. Die Formulierung der Substanzen erfolgt in Form einer 0.5%-igen Tylosesuspension mit 2% DMSO oder einer 0.5%-igen Tylosesuspension. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U/ 1.5 ml) aus dem Schwamm freigesetzt und in Gegenwart von MEM, 10% foetalem Kälberserum, 10% DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wird die Anzahl infizierter Zellen bzw. infektiöser Viruspartikel (infectious center assay) nach Substanzbehandlung im Vergleich zur placebobehandelten Kontrollgruppe.

### CY-P-Inhibitions-Assay

Zur Untersuchung der mechanism-based (irreversiblen) Inhibition von CYP3A4 wird die Testsubstanz in verschiedenen Konzentrationen mit Humanlebermikrosomen (2mg/ml mikrosomales Protein) in Kaliumphosphatpuffer pH 7.4 unter Zusatz von NADPH-generierendem System (NADP⁺, Glucose-6-phosphat und Glucose-6-phosphatdehydrogenase) bei 37°C inkubiert. Zu verschiedenen Zeitpunkten werden 2 Aliqouts aus der Inkubation entnommen.

Das erste Aliqout wird 1:50 in eine neue Inkubationslösung (Phosphatpuffer, NADPH-generierendes System und 10 µM Midazolam) für weitere 10 min bei 37°C inkubiert. Danach wird die Inkubation mit Acetonitril auf Eis gestoppt, in der Zentrifuge bei 15000g das Protein pelletiert, und der Überstand mit HPLC/MS nach Standardmethoden auf die Bildung von 1'-Hydroxymidazolam analysiert.

Das zweite Aliquot wird mit Acetonitril auf Eis gestoppt und mit HPLC/UV/MS auf verbleibenden Testsubstanz analysiert.

Aus beiden analytischen Datensätzen werden für irreversible Inhibition typische Parameter (k_{inact}, Kᵢ und partition ratio r) bestimmt und die Testsubstanz damit bewertet (vgl. A. Madan, et al., in A.D. Rodrigues (ed.) "Drug-Drug Interaction" in "Drugs and the Pharmaceutical Science", Vol. 116, , ISBN 0-8247-0283.2, Marcel Dekker Inc., New York, 2002.).

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

10-500 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 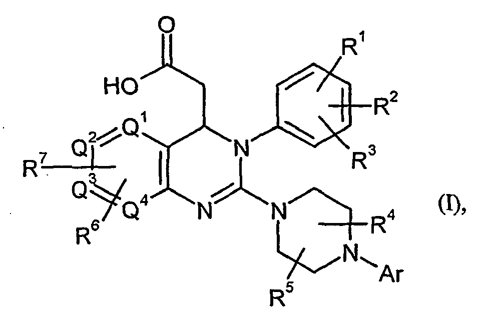 in welcher
Ar für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Formyl, Hydroxycarbonyl, Alkylcarbonyl, Alkoxycarbonyl, Trifluormethyl, Halogen, Cyano, Hydroxy, Amino, Alkylamino, Aminocarbonyl und Nitro,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,
oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls vorhandener dritter Substituent unabhängig davon aus der genannten Gruppe ausgewählt wird,
Q¹, Q², Q³ und Q⁴ für CH oder N stehen,
wobei ein oder zwei von Q¹, Q², Q³ und Q⁴ für N stehen und die anderen gleichzeitig für CH stehen,
R¹ für Wasserstoff, Amino, Alkyl, Alkoxy, Alkylamino, Alkylthio, Cyano, Halogen, Nitro oder Trifluormethyl steht,
R² für Wasserstoff, Alkyl, Alkoxy, Alkylthio, Cyano, Halogen, Nitro oder Trifluormethyl steht,
R³ für Amino, Alkyl, Alkoxy, Alkylamino, Alkylthio, Cyano, Halogen, Nitro, Trifluormethyl, Alkylsulfonyl oder Alkylaminosulfonyl steht
oder
einer der Reste R¹, R² und R³ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,
R⁴ für Wasserstoff oder Alkyl steht,
R⁵ für Wasserstoff oder Alkyl steht
oder
die Reste R⁴ und R⁵ im Piperazin-Ring an genau gegenüberliegenden Kohlenstoffatomen gebunden sind und eine gegebenenfalls mit 1 bis 2 Methylgruppen substituierte Methylen-Brücke bilden,
R⁶ für Wasserstoff, Alkyl, Alkoxy, Alkylthio, Formyl, Hydroxycarbonyl, Aminocarbonyl, Alkylcarbonyl, Alkoxycarbonyl, Trifluormethyl, Halogen, Cyano, Hydroxy oder Nitro steht
und
R⁷ für Wasserstoff, Alkyl, Alkoxy, Alkylthio, Formyl, Hydroxycarbonyl, Alkylcarbonyl, Alkoxycarbonyl, Trifluormethyl, Halogen, Cyano, Hydroxy oder Nitro steht,
oder eines ihrer Salze, ihrer Solvate oder der Solvate ihrer Salze.

2. Verbindung nach Anspruch 1, **dadurch gekennzeichnet, dass**
Ar für Phenyl steht, worin Phenyl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Hydroxycarbonyl, C₁-C₆-Alkylcarbonyl, C₁-C₆-Alkoxycarbonyl, Trifluormethyl, Fluor, Chlor, Brom, Cyano, Hydroxy, Amino, C₁-C₆-Alkylamino und Nitro, oder zwei der Substituenten am Phenyl zusammen mit den Kohlenstoffatomen, an die sie gebunden sind, ein 1,3-Dioxolan bilden und ein gegebenenfalls vorhandener dritter Substituent unabhängig davon aus der genannten Gruppe ausgewählt wird,
Q¹, Q² und Q³ für CH oder N stehen,
wobei immer genau einer von Q¹, Q² und Q³ für N steht und die anderen gleichzeitig für CH stehen,
Q⁴ für CH steht,
R¹ für Wasserstoff, C₁-C₃-Alkyl, C₁-C₃-Alkoxy, C₁-C₃-Alkylthio, Fluor oder Chlor steht,
R² für Wasserstoff, C₁-C₃-Alkyl, C₁-C₃-Alkoxy, C₁-C₃-Alkylthio, Fluor oder Chlor steht,
R³ für C₁-C₄-Alkyl, Cyano, Fluor, Chlor, Nitro, Trifluormethyl oder C₁-C₃-Alkyl-sulfonyl steht,
oder
einer der Reste R¹, R² und R³ für Wasserstoff, C₁-C₃-Alkyl, C₁-C₃-Alkoxy, Cyano, Halogen, Nitro oder Trifluormethyl steht und die anderen beiden zusammen mit den Kohlenstoffatomen, an die sie gebunden sind, einen Cyclopentan-Ring oder einen Cyclohexan-Ring bilden,
R⁴ für Wasserstoff oder Methyl steht,
R⁵ für Wasserstoff steht,
R⁶ für Wasserstoff, C₁-C₃-Alky), C₁-C₃-Alkoxy, Hydroxycarbonyl, Aminocarbonyl, Trifluormethyl, Fluor, Chlor, Cyano, Hydroxy oder Nitro steht
und
R⁷ für Wasserstoff, C₁-C₃-Alkyl, C₁-C₃-Alkoxy, Fluor, Chlor, Cyano oder Hydroxy steht.

3. Verbindung nach Anspruch 1 oder 2, **dadurch gekennzeichnet, dass**
Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
Q¹, und Q³ für CH oder N stehen,
wobei immer genau einer von Q¹, Q² und Q³ für N steht und die anderen gleichzeitig für CH stehen,
Q⁴ für CH steht,
R¹ für Wasserstoff, Methyl, Methoxy, Methylthio, Fluor oder Chlor steht,
R² für Wasserstoff steht,
R³ für Methyl, iso-Propyl, *tert*-Butyl, Cyano, Fluor, Chlor, Nitro oder Trifluormethyl steht,
R⁴ für Wasserstoff steht,
R⁵ für Wasserstoff steht,
R⁶ für Wasserstoff, Aminocarbonyl, Fluor, Chlor, Cyano oder Hydroxy steht
und
R⁷ für Wasserstoff steht.

4. Verbindung nach einem der Ansprüche 1 bis 3, **dadurch gekennzeichnet, dass**
Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
Q¹, Q² und Q³ für CH oder N stehen,
wobei immer genau einer von Q¹, Q² und Q³ für N steht und die anderen gleichzeitig für CH stehen,
Q⁴ für CH steht,
R¹ für Wasserstoff, Methyl oder Methoxy steht,
R² für Wasserstoff steht,
R³ für Methyl, *tert*-Butyl, Chlor oder Trifluormethyl steht,
R⁴ für Wasserstoff steht,
R⁵ für Wasserstoff steht,
R⁶ für Wasserstoff steht
und
R⁷ für Wasserstoff steht.

5. Verfahren zur Herstellung einer Verbindung der Formel (I) nach Anspruch 1, **dadurch gekennzeichnet, dass** eine Verbindung der Formel 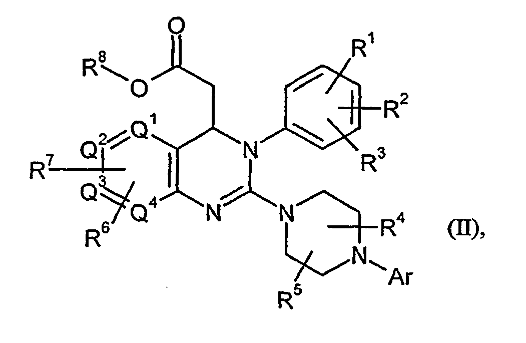 in welcher
Ar, Q¹, Q², Q³, Q⁴, R¹, R²,R³, R⁴, R⁵, R⁶ und R⁷ die in Anspruch 1 angegebene Bedeutung haben und
R⁸ für Alkyl, bevorzugt für Methyl oder Ethyl oder für *tert*-Butyl, steht,
mit einer Base oder einer Säure umgesetzt wird.

6. Verbindung nach einem der Ansprüche 1 bis 4 zur Behandlung und/oder Prophylaxe von Krankheiten.

7. Arzneimittel enthaltend eine Verbindung nach einem der Ansprüche 1 bis 4 in Kombination mit einem inerten, nichttoxischen, pharmazeutisch geeigneten Hilfsstoff

8. Verwendung einer Verbindung nach einem der Ansprüche 1 bis 4 zur Herstellung eines Arzneimittels zur Behandlung und/oder Prophylaxe von Virusinfektionen.

9. Verwendung nach Anspruch 8, **dadurch gekennzeichnet, dass** die Virusinfektion eine Infektion mit dem humanen Cytomegalovirus (HCMV) oder einem anderen Vertreter der Gruppe der Herpes viridae ist.

10. Arzneimittel nach Anspruch 7 zur Behandlung und/oder Prophylaxe von Virusinfektionen.

## Claims

1. Compound of the formula 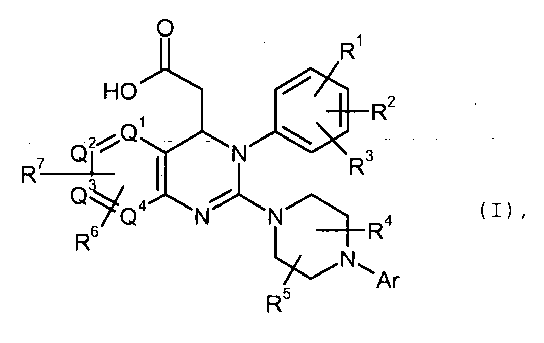 in which
Ar is aryl, in which aryl may be substituted by 1 to 3 substituents, where the substituents are selected independently of one another from the group consisting of alkyl, alkoxy, formyl, hydroxycarbonyl, alkylcarbonyl, alkoxycarbonyl, trifluoromethyl, halogen, cyano, hydroxy, amino, alkylamino, aminocarbonyl and nitro,
in which alkyl may be substituted by 1 to 3 substituents, where the substituents are selected independently of one another from the group consisting of halogen, amino, alkylamino, hydroxy and aryl,
or two of the substituents on the aryl form together with the carbon atoms to which they are bonded a 1,3-dioxolane, a cyclopentane ring or a cyclohexane ring, and an optionally present third substituent is selected independently thereof from the said group,
Q¹, Q², Q³ and Q⁴ are CH or N,
where one or two of Q¹, Q², Q³ and Q⁴ are N and the others are simultaneously CH,
R¹ is hydrogen, amino, alkyl, alkoxy, alkylamino, alkylthio, cyano, halogen, nitro or trifluoromethyl,
R² is hydrogen, alkyl, alkoxy, alkylthio, cyano, halogen, nitro or trifluoromethyl,
R³ is amino, alkyl, alkoxy, alkylamino, alkylthio, cyano, halogen, nitro, trifluoromethyl, alkylsulphonyl or alkylaminosulphonyl,
or
one of the radicals R¹, R² and R³ is hydrogen, alkyl, alkoxy, cyano, halogen, nitro or trifluoromethyl, and the other two form together with the carbon atoms to which they are bonded a 1,3-dioxolane, a cyclopentane ring or a cyclohexane ring,
R⁴ is hydrogen or alkyl,
R⁵ is hydrogen or alkyl,
or
the radicals R⁴ and R⁵ in the piperazine ring are bonded to exactly opposite carbon atoms and form a methylene bridge optionally substituted by 1 to 2 methyl groups,
R⁶ is hydrogen, alkyl, alkoxy, alkylthio, formyl, hydroxycarbonyl, aminocarbonyl, alkylcarbonyl, alkoxycarbonyl, trifluoromethyl, halogen, cyano, hydroxy or nitro,
and
R⁷ is hydrogen, alkyl, alkoxy, alkylthio, formyl, hydroxycarbonyl, alkylcarbonyl, alkoxycarbonyl, trifluoromethyl, halogen, cyano, hydroxy or nitro,
or one of the salts thereof, the solvates thereof or the solvates of the salts thereof.

2. Compound according to Claim 1, **characterized in that**
Ar is phenyl, in which phenyl may be substituted by 1 to 3 substituents, where the substituents are selected independently of one another from the group consisting of C₁-C₆-alkyl, C₁-C₆-alkoxy, hydroxycarbonyl, C₁-C₆-alkylcarbonyl, C₁-C₆-alkoxycarbonyl, trifluoromethyl, fluorine, chlorine, bromine, cyano, hydroxy, amino, C₁-C₆-alkylamino and nitro,
or two of the substituents on the phenyl form together with the carbon atoms to which they are bonded a 1,3-dioxolane, and an optionally present third substituent is selected independently thereof from the said group,
Q¹, Q² and Q³ are CH or N,
where always exactly one of Q¹, Q² and Q³ is N and the others are simultaneously CH,
Q⁴ is CH,
R¹ is hydrogen, C₁-C₃-alkyl, C₁-C₃-alkoxy, C₁-C₃-alkylthio, fluorine or chlorine,
R² is hydrogen, C₁-C₃-alkyl, C₁-C₃-alkoxy, C₁-C₃-alkylthio, fluorine or chlorine,
R³ is C₁-C₄-alkyl, cyano, fluorine, chlorine, nitro, trifluoromethyl or C₁-C₃-alkylsulphonyl,
or
one of the radicals R¹, R² and R³ is hydrogen, C₁-C₃-alkyl, C₁-C₃-alkoxy, cyano, halogen, nitro or trifluoromethyl, and the other two form together with the carbon atoms to which they are bonded a cyclopentane ring or a cyclohexane ring,
R⁴ is hydrogen or methyl,
R⁵ is hydrogen,
R⁶ is hydrogen, C₁-C₃-alkyl, C₁-C₃-alkoxy, hydroxycarbonyl, aminocarbonyl, trifluoromethyl, fluorine, chlorine, cyano, hydroxy or nitro,
and
R⁷ is hydrogen, C₁-C₃-alkyl, C₁-C₃-alkoxy, fluorine, chlorine, cyano or hydroxy.

3. Compound according to Claim 1 or 2, **characterized in that**
Ar is phenyl, in which phenyl may be substituted by 1 to 2 substituents, where the substituents are selected independently of one another from the group consisting of methyl, methoxy, fluorine and chlorine,
Q¹ , Q² and Q³ are CH or N,
where always exactly one of Q¹, Q² and Q³ is N, and the others are simultaneously CH,
Q⁴ is CH,
R¹ is hydrogen, methyl or methoxy, methylthio, fluorine or chlorine,
R² is hydrogen,
R³ is methyl, isopropyl, *tert*-butyl, cyano, fluorine chlorine, nitro or trifluoromethyl,
R⁴ is hydrogen,
R⁵ is hydrogen,
R⁶ is hydrogen, aminocarbonyl, fluorine, chlorine, cyano or hydroxy,
and
R⁷ is hydrogen.

4. Compound according to any of Claims 1 to 3, **characterized in that**
Ar is phenyl, in which phenyl may be substituted by 1 to 2 substituents, where the substituents are selected independently of one another from the group consisting of methyl, methoxy, fluorine and chlorine,
Q¹, Q² and Q³ are CH or N,
where always exactly one of Q¹, Q² and Q³ is N, and the others are simultaneously CH,
Q⁴ is CH,
R¹ is hydrogen, methyl or methoxy,
R² is hydrogen,
R³ is methyl, *tert*-butyl, chlorine or trifluoromethyl,
R⁴ is hydrogen,
R⁵ is hydrogen,
R⁶ is hydrogen
and
R⁷ is hydrogen.

5. Process for preparing a compound of the formula (I) according to Claim 1, **characterized in that** a compound of the formula 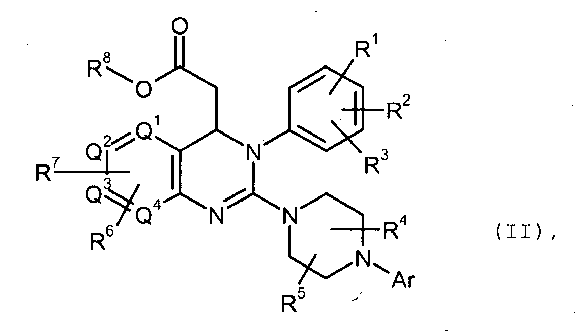 in which
Ar, Q¹, Q², Q³, Q⁴, R¹, R², R³, R⁴, R⁵, R⁶ and R⁷ have the meaning indicated in Claim 1, and
R⁸ is alkyl, preferably methyl or ethyl, or *tert-*butyl,
is reacted with a base or an acid.

6. Compound according to any of Claims 1 to 4 for the treatment and/or prophylaxis of diseases.

7. Medicament comprising a compound according to any of Claims 1 to 4 in combination with an inert, non-toxic, pharmaceutically suitable excipient.

8. Use of a compound according to any of Claims 1 to 4 for producing a medicament for the treatment and/or prophylaxis of viral infections.

9. Use according to Claim 8, **characterized in that** the viral infection is an infection with human cytomegalovirus (HCMV) or another representative of the group of Herpes viridae.

10. Medicament according to Claim 7 for the treatment and/or prophylaxis of viral infections.

## Revendications

1. Composé de formule 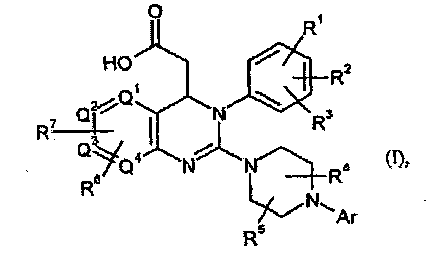 dans laquelle
Ar représente un reste aryle, ce reste aryle pouvant être substitué avec 1 à 3 substituants, les substituants étant choisis indépendamment les uns des autres dans le groupe constitué d'alkyle, alkoxy, formyle, hydroxycarbonyle, alkylcarbonyle, alkoxycarbonyle, trifluorométhyle, halogéno, cyano, hydroxy, amino, alkylamino, aminocarbonyle et nitro,
où le radical alkyle peut être substitué avec 1 à 3 substituants, les substituants étant choisis indépendamment les uns des autres dans le groupe constitué d'halogéno, amino, alkylamino, hydroxy et aryle,
ou bien deux des substituants portés par le reste aryle forment, conjointement avec les atomes de carbone auxquels ils sont liés, un 1,3-dioxolanne, un noyau cyclopentane ou un noyau cyclohexane, et un troisième substituant éventuellement présent est choisi, indépendamment de cela, dans le groupe mentionné,
Q¹, Q², Q³ et Q⁴ représentent CH ou N, l'un ou deux de Q¹, Q², Q³ et Q⁴ représentant N et les autres représentant simultanément CH,
R¹ représente l'hydrogène, un reste amino, alkyle, alkoxy, alkylamino, alkylthio, cyano, halogéno, nitro ou trifluorométhyle,
R² représente l'hydrogène, un reste alkyle, alkoxy, alkylthio, cyano, halogéno, nitro ou trifluorométhyle,
R³ représente un reste amino, alkyle, alkoxy, alkylamino, alkylthio, cyano, halogéno, nitro, trifluorométhyle, alkylsulfonyle ou alkylaminosulfonyle,
ou bien
l'un des restes R¹, R² et R³ représente l'hydrogène, un reste alkyle, alkoxy, cyano, halogéno, nitro ou trifluorométhyle et les deux autres forment, conjointement avec les atomes de carbone auxquels ils sont liés, un 1,3-dioxolanne, un noyau cyclopentane ou un noyau cyclohexane,
R⁴ représente l'hydrogène ou un reste alkyle,
R⁵ représente l'hydrogène ou un reste alkyle,
ou bien,
les restes R⁴ et R⁵ dans le noyau pipérazine sont liés à des atomes de carbone exactement opposés et forment un pont méthylène éventuellement substitué avec 1 ou 2 groupes méthyle,
R⁶ représente l'hydrogène, un reste alkyle, alkoxy, alkylthio, formyle, hydroxycarbonyle, aminocarbonyle, alkylcarbonyle, alkoxycarbonyle, trifluorométhyle, halogéno, cyano, hydroxy ou nitro
et
R⁷ représente l'hydrogène, un reste alkyle, alkoxy, alkylthio, formyle, hydroxycarbonyle, alkylcarbonyle, alkoxycarbonyle, trifluorométhyle, halogéno, cyano, hydroxy ou nitro,
ou l'un de ses sels, de ses produits de solvatation ou des produits de solvatation de ses sels.

2. Composé suivant la revendication 1, **caractérisé en ce que**
Ar représente un reste phényle, ce reste phényle pouvant être substitué avec 1 à 3 substituants, les substituants étant choisis indépendamment les uns des autres dans le groupe constitué des substituants alkyle en C₁ à C₆, alkoxy en C₁ à C₆, hydroxycarbonyle, (alkyle en C₁ à C₆)carbonyle, (alkoxy en C₁ à C₆)carbonyle, trifluorométhyle, fluoro, chloro, bromo, cyano, hydroxy, amino, alkylamino en C₁ à C₆ et nitro,
ou bien deux des substituants portés par le reste phényle forment un 1,3-dioxolanne conjointement avec les atomes de carbone auxquels ils sont liés, et un troisième substituant éventuellement présent est choisi, indépendamment de cela, dans le groupe mentionné,
Q¹, Q² et Q³ représentent CH ou N, dans tous les cas, précisément l'un de Q¹, Q² et Q³ représentant N et les deux autres représentant simultanément CH,
Q⁴ représente CH,
R¹ représente l'hydrogène, un reste alkyle en C₁ à C₃, alkoxy en C₁ à C₃, alkylthio en C₁ à C₃, fluoro ou chloro,
R² représente l'hydrogène, un reste alkyle en C₁ à C₃, alkoxy en C₁ à C₃, alkylthio en C₁ à C₃, fluoro ou chloro,
R³ représente un reste alkyle en C₁ à C₄, cyano, fluoro, chloro, nitro, trifluorométhyle ou alkylsulfonyle en C₁ à C₃,
ou bien,
l'un des restes R¹, R² et R³ représente l'hydrogène, un reste alkyle en C₁ à C₃, alkoxy en C₁ à C₃, cyano, halogéno, nitro ou trifluorométhyle et les deux autres forment, conjointement avec les atomes de carbone auxquels ils ont liés, un noyau cyclopentane ou un noyau cyclohexane,
R⁴ représente l'hydrogène ou un reste méthyle,
R⁵ représente l'hydrogène,
R⁶ représente l'hydrogène, un reste alkyle en C₁ à C₃, alkoxy en C₁ à C₃, hydroxycarbonyle, aminocarbonyle, trifluorométhyle, fluoro, chloro, cyano, hydroxy ou nitro
et
R⁷ représente l'hydrogène, un reste alkyle en C₁ à C₃, alkoxy en C₁ à C₃, fluoro, chloro, cyano ou hydroxy.

3. Composé suivant la revendication 1 ou 2, **caractérisé en ce que**
Ar représente un reste phényle, ce reste phényle pouvant être substitué avec 1 ou 2 substituants, les substituants étant choisis indépendamment l'un de l'autre dans le groupe constitué des substituants méthyle, méthoxy, fluoro et chloro
Q¹, Q² et Q³ représentent CH ou N, dans tous les cas, précisément l'un de Q¹, Q² et Q³ représentant N et les autres représentant simultanément CH,
Q⁴ représente CH,
R¹ représente l'hydrogène, un reste méthyle, méthoxy, méthylthio, fluoro ou chloro,
R² représente l'hydrogène,
R³ représente un reste méthyle, isopropyle, tertiobutyle, cyano, fluoro, chloro, nitro ou trifluorométhyle,
R⁴ représente l'hydrogène,
R⁵ représente l'hydrogène,
R⁶ représente l'hydrogène, un reste aminocarbonyle, fluoro, chloro, cyano ou hydroxy,
et
R⁷ représente l'hydrogène.

4. Composé suivant l'une des revendications 1 à 3, **caractérisé en ce que**
Ar représente un reste phényle, ce reste phényle pouvant être substitué avec 1 ou 2 substituants, les substituants étant choisis indépendamment l'un de l'autre dans le groupe constitué des substituants méthyle, méthoxy, fluoro et chloro
Q¹, Q² et Q³ représentent CH ou N, dans tous les cas, précisément l'un de Q¹, Q² et Q³ représentant N et les autres représentant simultanément CH,
Q⁴ représente CH,
R¹ représente l'hydrogène, un reste méthyle ou méthoxy,
R² représeente l'hydrogène,
R³ représente un reste méthyle, tertiobutyle, chloro ou trifluorométhyle,
R⁴ représente l'hydrogène,
R⁵ représente l'hydrogène,
R⁶ représente l'hydrogène
et
R⁷ représente l'hydrogène.

5. Procédé de production d'un composé de formule (I) suivant la revendication 1, **caractérisé en ce qu'**un composé de formule 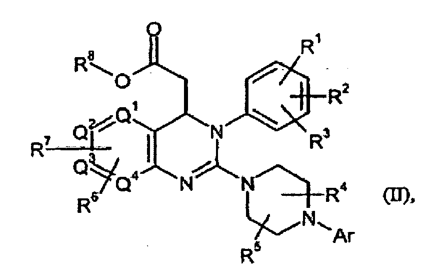 dans laquelle
Ar, Q¹, Q², Q³, Q⁴, R¹, R², R³, R⁴, R⁵, R⁶ et R⁷ ont la définition indiquée dans la revendication 1 et
R⁸ représente un reste alkyle, préférentiellement un reste méthyle ou éthyle ou un reste tertiobutyle, est amené à réagir avec une base ou avec un acide.

6. Composé suivant l'une des revendications 1 à 4, destiné au traitement et/ou à la prophylaxie de maladies.

7. Médicament contenant un composé suivant l'une des revendications 1 à 4 en combinaison avec une substance auxiliaire inerte non toxique pharmaceutiquement acceptable.

8. Utilisation d'un composé suivant l'une des revendications 1 à 4 pour la préparation d'un médicament destiné au traitement et/ou à la prophylaxie d'infections virales.

9. Utilisation suivant la revendication 8, **caractérisée en ce que** l'infection virale est une infection causée par le cytomégalovirus humain (HCMV) ou par un autre virus du groupe des Herpes viridae.

10. Médicament suivant la revendication 7, destiné au traitement et/ou à la prophylaxie d'infections virales.
